# EUROPEAN PATENT SPECIFICATION

(11) **EP 2 094 726 B1**
(45) Date of publication and mention of the grant of the patent: **08.06.2016**
(21) Application number: 07870874.0
(22) Date of filing: 15.11.2007
(51) Int. Cl.: C07K 14/705, C07K 16/00, C07K 16/28, C07H 21/04, C12P 21/02, A61K 38/00, A61K 39/00, A61P 29/00

(54) **COMPOSITIONS AND METHODS RELATED TO TOLL-LIKE RECEPTOR-3**
ZUSAMMENSETZUNGEN UND VERFAHREN IN ZUSAMMENHANG MIT DEM TOLL-LIKE-REZEPTOR 3
COMPOSITIONS ET PROCÉDÉS ASSOCIÉS AU RÉCEPTEUR-3 DE TYPE TOLL

(30) Priority: 15.11.2006 US 859085 P
(43) Date of publication of application: 02.09.2009
(73) Proprietor: THE TEXAS A&M UNIVERSITY SYSTEM, College Station, TX 77843-3369 (US)
(72) Inventor: KAO, Cheng C., College Station, TX 77840 (US); THARACHAPARAMBA, Ranjith Kumar, College Station, TX 77843 (US)
(74) Representative: Glawe, Delfs, Moll
(86) International application number: PCT/US2007/023825
(87) International publication number: WO 2008/063493

(56) References cited:
- WO-A1-03/022296
- WO-A2-2006/111946
- US-A1- 2003 022 302
- US-A1- 2003 034 090
- DE BOUTEILLER ODETTE ET AL: "Recognition of double-stranded RNA by human toll-like receptor 3 and downstream receptor signaling requires multimerization and an acidic pH", JOURNAL OF BIOLOGICAL CHEMISTRY, THE AMERICAN SOCIETY OF BIOLOGICAL CHEMISTS, INC, US, vol. 280, no. 46, 6 September 2005 (2005-09-06), pages 38133-38145, XP002522758, ISSN: 0021-9258, DOI: 10.1074/JBC.M507163200 [retrieved on 2005-09-06]
- ALEXOPOULOU L ET AL: "Recognition of double-stranded RNA and activation of NF-kB by toll-like receptor 3", NATURE: INTERNATIONAL WEEKLY JOURNAL OF SCIENCE, NATURE PUBLISHING GROUP, UNITED KINGDOM, vol. 413, 18 October 2001 (2001-10-18), pages 732-738, XP002968529, ISSN: 0028-0836, DOI: 10.1038/35099560
- C. T. RANJITH-KUMAR ET AL: "Effects of Single Nucleotide Polymorphisms on Toll-like Receptor 3 Activity and Expression in Cultured Cells", JOURNAL OF BIOLOGICAL CHEMISTRY, vol. 282, no. 24, 1 June 2007 (2007-06-01) , pages 17696-17705, XP055018029, ISSN: 0021-9258, DOI: 10.1074/jbc.M700209200
- RANJITH-KUMAR C T ET AL: "Biochemical and functional analyses of the human Toll-like receptor 3 ectodomain", JOURNAL OF BIOLOGICAL CHEMISTRY 20070302 AMERICAN SOCIETY FOR BIOCHEMISTRY AND MOLECULAR BIOLOGY INC. US,, vol. 282, no. 10, 2 March 2007 (2007-03-02), pages 7668-7678, XP007914953,
- ZHANG SHEN-YING ET AL: "TLR3 deficiency in patients with herpes simplex encephalitis", SCIENCE (WASHINGTON D C), vol. 317, no. 5844, September 2007 (2007-09), pages 1522-1527, XP002668826, ISSN: 0036-8075

## Description

### FIELD OF INVENTION

The invention relates to compositions and methods related to Toll-like receptor (TLR) polypeptides. The invention relates to managing TLR3 related diseases. The invention relates to methods of preventing and treating inflammation. In some embodiments, the invention relates to antagonists of TLR3, to amino acid sequences that act as dominant negative molecules, and to nucleic acid sequences that encode said amino acid sequences. The invention relates to the manipulation of biological materials to evaluate TLR3 activity.

### BACKGROUND OF INVENTION

Toll-like receptor 3 (TLR3) has been shown to be involved in inflammation processes. Inflammation is the body's protective response to an injury that is caused by cytokines. Overstimulation of the inflammation response is a factor in a variety of inflammatory diseases. For example, inflammation of the joints is associated with rheumatoid arthritis. Inflammation of the small tubes that transport air to the lungs is associated with asthma. Non-steroidal anti-inflammatory drugs may be used to treat these symptoms. However, these drugs often have varying success as well as adverse side effects. Thus, there is a need to identify compositions and methods for managing inflammatory responses that have limited adverse affects.

### THE CLAIMED SUBJECT-MATTER VIS-À-VIS THE CITED PRIOR ART

WO 03/022296 A1 discloses treating autoimmune diseases with compounds that inhibit the formation of autoimmune complexes or inhibit the interaction of autoimmune complexes with the TLR. DE BOUTEILER ODETTE et al.: "Recognition of double-stranded RNA by human toll-like receptor 3 and downstream receptor signalling requires multimerization and an acidic pH", JOURNAL OF BIOLOGICAL CHEMISTRY, THE AMERICAN SOCIETY OF BIOLOGICAL CHEMISTS, INC, US, vol. 280, no. 46, September 6, 2005, pages 38133-38145 discloses chimeric molecules in which the amino terminal domain of TLR3 is fused to the transmembrane and intracellular carboxyl terminus of CD32a. ALEXOPOULOU L et al.: "Recognition of double-stranded RNA and activation of NF-kB by toll-like receptor 3", NATURE: INTERNATIONAL WEEKLY JOURNAL OF SCIENCE, NATURE PUBLISHING GROUP, UNITED KINGDOM, vol. 413, October 18, 2001, pages 732-738 discloses complete TLR3 mutants. None of these references mention mutations within Loop 2 of the TLR3 ectodomain as defined in the appended claims.

### SUMMARY OF THE INVENTION

The invention is set out in the appended claims. The embodiments of the description which do not fall within the scope of said claims are provided for illustrative purposes only and do not form part of the present invention.

The invention relates to compositions and methods related to Toll-like receptor (TLR) polypeptides. The invention relates to managing TLR3 related diseases. The invention relates to methods of preventing and treating inflammation. In some embodiments, the invention relates to antagonists of TLR3, to amino acid sequences that act as dominant negative molecules, and to nucleic acid sequences that encode said amino acid sequences. The invention relates to the manipulation of biological materials to evaluate TLR3 activity.

In some embodiments, the invention relates to a pharmaceutical composition comprising an amino acid sequence that is a TLR3 mutant having a mutation in Loop2 and that is a dominant negative inhibitor of a TLR polypeptide. In further embodiments, said inhibited TLR is wild-type TLR3 or TLR9. Said TLR3 may comprise C and N-terminal motifs capable of disulfide bond formation. The mutant is in Loop2 within TLR3. In further embodiments, the amino acid sequence comprises the motif HANPGGIY.

TLR3 mutant may interact with other TLRs.

The invention relates to a composition comprising a polypeptide mutant of SEQ ID NO.:1. Said mutant may be one or more selected from the group consisting of C242A, C356A, C28A, C37A, C37S, C37M, C95A, C122A, C122S, C122M, C649A, C649S, C649M, C651A, C696A, C696S, C696M, R65A, K89A, K117A, K137A, K139A, K147A, K163A, K210A, R331A, R394A, K418A, K493A, K589A, K613A, K627A, R635A, R643A, H539E, N541A, N466A, R489A, N515A, N516A, N517A, N540A, R544A, N572A, E442A, E442D, E442K, K467A, K467E, K547A, and D575A. Said mutant may be a dominant negative inhibitor of a TLR. Said mutant may be a dominant negative inhibitor of wild-type TLR3 or TLR9.

The invention relates to a pharmaceutical composition comprising an antibody to TLR3. The antibody may be to Loop2. The antibody may be humanized.

The invention relates to a TLR3 polypeptide comprising a tag in Loop1. Said tag may comprise a cysteine amino acid. Said tag may be complexed with arsenic containing fluorophores.

The invention relates to a pharmaceutical composition comprising an amino acid sequence with a substituted or unsubstituted motif X¹X²NX⁴GGPX⁸X⁹ wherein X¹, X², X⁴, X⁸, and X⁹ are each individually and independently a naturally or nonnaturally occurring amino acid. In some examples, X¹ is H, R, or N; X² is A or N; X⁴ is P or V; X⁸ is I or V; and X⁹ is Y, H, Q, N, and M. In further examples, said amino acid sequence is less than 500, 400, 300, 200, 100, 50, or 25 residues.

In additional embodiments, the invention relates to a pharmaceutical composition comprising a dominant negative TLR3 polypeptide comprising a mutation in Loop2 for a medical use, in particular for use as a medicament for preventing or treating an inflammatory disease. In further embodiments, said inflammatory disease is selected from the group consisting of pulmonary diseases, autoimmune diseases, fibrotic diseases, and kidney diseases. In further embodiments, said pulmonary disease is selected form the group consisting of asthma, asthma exacerbation, microbial-associated pneumonia, sarcoidosis and cystic fibrosis. In further embodiments, said autoimmune disease is selected from the group consisting of rheumatoid arthritis, psoriatic arthritis, and giant cell arteritis. In further embodiments, said kidney disease is lupus nephritis. In further embodiments, said fibrotic disease is liver fibrosis. Said dominant negative TLR3 molecule may be an amino acid sequence with the substituted or unsubstituted motif X¹X²NX⁴GGPX⁸X⁹ wherein X¹, X², X⁴, X⁸, and X⁹ are each individually and independently a naturally or nonnaturally occurring amino acid. In further examples, X¹ is H, R, or N; X² is A or N; X⁴ is P or V; X⁸ is I or V; and X⁹ is Y, H, Q, N, and M. In further examples, said amino acid sequence is less than 500 residues. In further embodiments, the composition may be for administration with a second therapeutic agent. In further embodiments, said second therapeutic agent is selected from the group consisting of antimicrobial agents, corticosteroids and

immuno-modulatory agents. In further embodiments, said antimicrobial agent is selected from the group consisting of an antibacterial agent, antiviral agents, antifungal agents, and antiparasitic agent. In further embodiments, said immuno-modulatory agent is selected from the group consisting of interferon gamma-1b, IFN-gamma, Actimmune, Tysabri, Natalizumab, Xolair, Omalizumab, Neulasta, Pegfilgrastim, Neupogen, Filgrastim, Anakinra, Humira, Adalimumab, Enbrel, TNF, Etanercept, Alefacept, Remicade, Infliximab, Raptiva, Efalizumab, Thymoglobulin, Infergen, Interferon, Muromaonab, Zenapax, Daclizumab, and Basiliximab. In further embodiments, said corticosteroid is selected from the group consisting of dexamethasone (Decadron), hydrocortisone, methylprednisolone (Medrol), prednisone, cortisone, betamethasone, and prednisolone. In further embodiments, said antibacterial agent is selected from the group consisting of sulfanilamide, Trimethoprim penicillin G, cephalexin, cefaclor, cefixime, meropenem, ertapenem, chlortetracycline, oxytetracycline erythromycin, azithromycin, and clarithromycin, clindamycin, quinupristin/dalfopristin, Ciprofloxacin Spectinomycin, Vancomycin, linezolid, and daptomycin. In further embodiments, said antiviral is selected from the group consisting of bacavir, acyclovir, agenerase, amatadine, amprenavir, crixivan, delavirdine, denavir, didanosine, efavirenz, epivir, famciclovir, famvir, fortovase, hivid, indinavir, ribavirin, invirase, lamivudine, nelfinavir, nevirapine, norvir, oseltamivir, penciclovir, relenza, rescriptor, retrovir, ritonavir, saquinavir, stavudine, sustiva, symdine, symmetrel, tamiflu, valacyclovir, valtrex, videx, viracept, viramune, zalcitabine, zerit, ziagen, zidovudine, zovirax, and zanamivir. In further embodiments, said antifungal agent is selected from the group consisting of nystatin, clotrimazole, econazole, ciclopirox olamine, ketoconazole, miconazole, terbinafine, and tolciclate. In further embodiments, said administration is selected from the group consisting of subcutaneous, oral, intravenous, intradermal, and intranasal routes.

In additional embodiments, the invention relates to a pharmaceutical composition comprising a nucleic acid sequence that encodes a dominant negative TLR3 amino acid sequence comprising a mutation in Loop2 for use as a medicament for preventing or treating inflammatory diseases. In further embodiments, said
inflammatory disease is selected from the group consisting of pulmonary diseases, autoimmune diseases, fibrotic diseases, and kidney diseases. In further embodiments, said pulmonary disease is selected from the group consisting of asthma, asthma exacerbation, microbial-associated pneumonia, sarcoidosis and cystic fibrosis. In further embodiments, said autoimmune disease is selected from the group consisting of rheumatoid arthritis, psoriatic arthritis, and giant cell arteritis. In further embodiments, said kidney disease is lupus nephritis. In further embodiments, said fibrotic disease is liver fibrosis. In further examples, said dominant negative TLR3 amino acid sequence is an amino acid sequence with the substituted or unsubstituted motif X¹X²NX⁴GGPX⁸X⁹ wherein X¹, X², X⁴, X⁸, and X⁹ are each individually and independently a naturally or nonnaturally occurring amino acid. In further examples, X¹ is H, R, or N; X² is A or N; X⁴ is P or V; X⁸ is I or V; and X⁹ is Y, H, Q, N, and M. In further examples, said amino acid sequence is less than 500 residues. In further embodiments, the composition further comprises administering a second therapeutic agent. In further embodiments, said second therapeutic agent is selected from the group consisting of antimicrobial agents, corticosteroids and immuno-modulatory agents. In further embodiments, said antimicrobial agent is selected from the group consisting of antibacterial agents, antiviral agents, antifungal agents, and antiparasitic agents. In further embodiments, said immuno-modulatory agent is selected from the group consisting of interferon gamma-1b, IFN-gamma, Actimmune, Tysabri, Natalizumab, Xolair, omalizumab, Neulasta, Pegfilgrastim, Neupogen, Filgrastim, Anakinra, Humira, Adalimumab, Enbrel, TNF, Etanercept, Alefacept, Remicade, infliximab, Raptiva, Efalizumab, Thymoglobulin, Infergen, Interferon, Muromaonab, Zenapax, Daclizumab, and Basiliximab. In further embodiments, said corticosteroid is selected from the group consisting of dexamethasone (Decadron), hydrocortisone, methylprednisolone (Medrol), prednisone, cortisone, betamethasone, and prednisolone. In further embodiments, said antibacterial agent is selected from the group consisting of sulfanilamide, Trimethoprim penicillin G, cephalexin, cefaclor, cefixime, meropenem, ertapenem, chlortetracycline, oxytetracycline erythromycin, azithromycin, and clarithromycin, clindamycin, quinupristin and dalfopristin, Ciprofloxacin Spectinomycin, Vancomycin, linezolid, and daptomycin. In further embodiments, said antiviral agent is selected from the group
consisting of bacavir, acyclovir, agenerase, amatadine, amprenavir, crixivan, delavirdine, denavir, didanosine, efavirenz, epivir, famciclovir, famvir, fortovase, hivid, indinavir, ribavirin, invirase, lamivudine, nelfinavir, nevirapine, norvir, oseltamivir, penciclovir, relenza, rescriptor, retrovir, ritonavir, saquinavir, stavudine, sustiva, symdine, symmetrel, tamiflu, valacyclovir, valtrex, videx, viracept, viramune, zalcitabine, zerit, ziagen, zidovudine, zovirax, and zanamivir. In further embodiments, said antifungal agent is selected from the group consisting of nystatin, clotrimazole, econazole, ciclopirox olamine, ketoconazole, miconazole, terbinafine, and tolciclate. In further embodiments, said administration is selected from the group consisting of subcutaneous, oral, intravenous, intradermal, and intranasal routes.

In some embodiments, the invention relates to a method of inhibiting Toll-like receptor 3 activity comprising: i) providing; a) a cell comprising TLR3 and b) a dominant negative TLR3 amino acid sequence comprising a mutation in Loop2, and ii) mixing said cell and said amino acid sequence in vitro under conditions such that TLR3 activity is inhibited. In further embodiments, said amino acid sequence is a mutant of an amino acid sequence with a substituted or unsubstituted motif X¹X²NX⁴GGPX⁸X⁹ wherein X¹, X², X⁴, X⁸, and X⁹ are each individually and independently a naturally or nonnaturally occurring amino acid. In further embodiments, X¹ is H, R, or N; X² is A or N; X⁴ is P or V; X⁸ is I or V; and X⁹ is Y, H, Q, N, and M. In further embodiments, said cells are selected from the group HEK, HeLa, COS, and Chinese Hamster Ovary cells.

The invention relates to a method of diagnosing a TLR3 related disease comprising: a) providing; i) a subject having cells that encode TLR3 and ii) a composition comprising a nucleic acid sequence encoding a nucleic acid sequence disclosed herein; b) mixing said cells and said nucleic acid sequence under conditions such that said TLR3 activity is measured. Said measured activity may be inhibited. Said cells may be selected from the group consisting of lung cells, kidney cells, and synovial fibroblasts. Said nucleic acid sequence may be wild-type TLR3 or a dominant negative inhibitor of wild-type TLR3.

The invention relates to pharmaceutical compositions comprising a non-steroidal anti-inflammatory compound and amino acid sequences disclosed herein.

The invention relates use of a dominant negative inhibitor of wild-type T for the manufacture of a medicament for the management of diseases disclosed herein.

The invention relates to the use of composition disclosed herein, including mutant TLR molecules as a reagent to identify molecule inhibitors by screening for binding. Said screening may involve correlating relative binding between the wild-type TLR and mutant TLR polypeptides. The mutant TLR molecule may be used in a cell-based assay for modular activity for cytokine productions. The mutant TLR molecule may be administered to a live subject.

### BRIEF DESCRIPTION OF THE SEVERAL VIEWS OF THE DRAWINGS

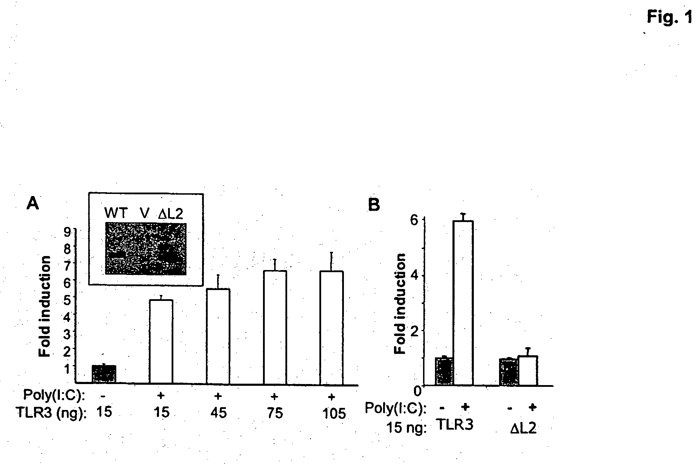

Figure 1 shows a cell-based assay to detect TLR3 activity. A) Effects of increasing concentration of plasmid expressing wild-type TLR3 on the activation of luciferase reporter activity in HEK 293T cells. The luciferase activity is expressed as the ratio of the firefly luciferase driven from promoter containing NF-κβ elements over the activity of the Renilla luciferase driven from the herpevirus thymidine kinase promoter. Activation of the firefly luciferase activity requires poly(I:C), added at 2.5 µg/ml of cell culture. Inset, a demonstration that HEK293 cells does not express endogenous level of TLR3. B) The TLR3 cell-based assay can detect a range of luciferase activity, from the higher levels seen with wild-type TLR3 and a mutant that has a deletion of an internal loop in TLR3.

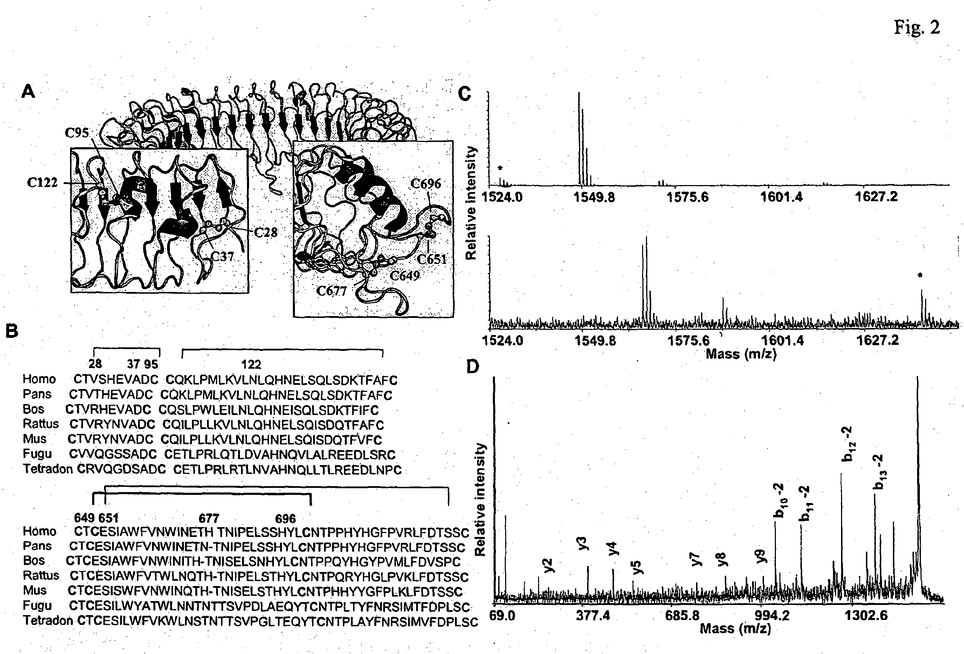

Figure 2 shows detection of disulfide bonds in the 3ECD. A) A 3ECD structure (PDB id 2A0Z) showing the residues near the N- and C-terminal portions of the 3ECD, which participate in disulfide bond formation. B) Sequence analysis of the cysteines in TLR3 ECDs from species across a wide phylogenetic range. The cysteine pairs involved in disulfide bond formation are indicated by the brackets. C) Mass spectra of a tryptic digest of TLR3 protein focusing on the m/z region containing the CTVSHEVADCSHLK
   peptide. The top spectrum is of the peptide containing the disulfide bond. The bottom spectrum is the reduced and alkylated form of the peptide. D) Tandem mass spectrometry analysis of the above peptide to confirm the assigned peptide sequence.

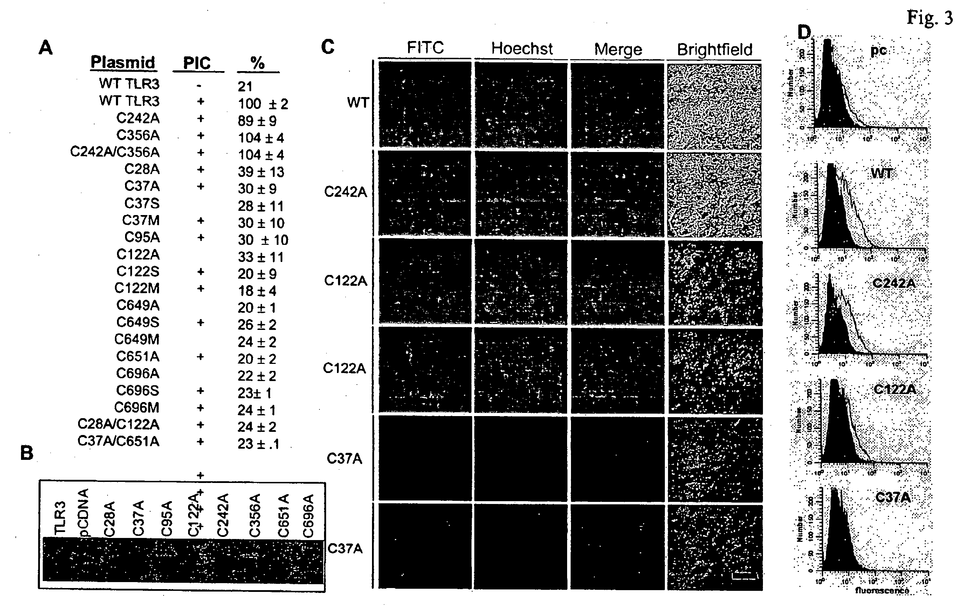

Figure 3 shows the effects of the mutations in the cysteines involved in the disulfide bond formation. A) A summary of the effects of mutations on TLR3 activity. All mutant names contain the amino acid, their position in TLR3 and the residue to which they were mutated. All activities are normalized to the wild-type TLR3 assayed in the same experiment. B) Western blot analysis of select mutants to analyze whether the mutation affects the expression of the protein. pCDNA is the plasmid vector used to express TLR3 or mutant TLR3s. C) In situ localization of TLR3 stained with FITC-labeled monoclonal antibody specific for TLR3. The names of the samples are shown on the left, the types of image taken are shown above the micrographs. D) Results of FACS analysis of several cysteine mutants looking at the cell-surface fluorescence. The monoclonal antibody recognizing TLR3, TLR3.7, is from eBioSciences Inc. (San Diego, CA). The distribution of the cells with the fluorescence intensity denoted on the horizontal axis is graphed. The shaded regions are signals from an immunoglobulin isotype of the monoclonal antibody recognizing TLR3. 'pc' corresponds to pcDNA and 'WT' to wild-type TLR3.

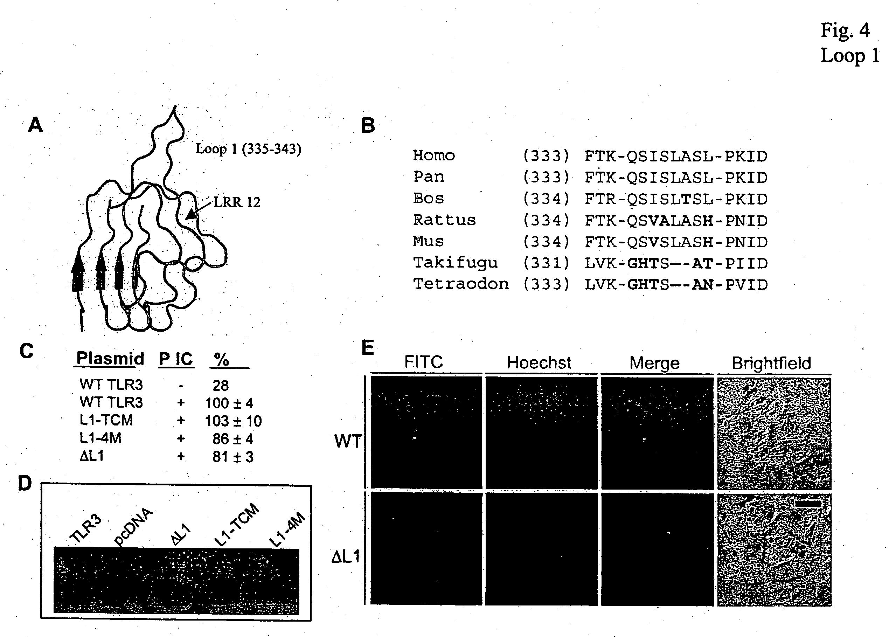

Figure 4 shows data in examination of the role of Loop1 on TLR3 function. A) A partial model of TLR3 showing the relative location of Loop1. B) A comparison of the sequences in Loop1 from different species. The residues that are different from the human Loop1 sequence are shown in bold. The residues that are apparently deleted are shown as a dash. C) Activity assay of wild-type TLR3 and mutations in Loop1 of TLR3. D) Western blot analysis examining whether the mutations in Loop1 affect protein expression. E) In situ localization of the mutant deleted for Loop1 in comparison to WT. The presence of TLR3 in punctate spots, the nuclei, and merging of the two results are shown as identified above the micrographs. The bar in the lower micrograph represents 20 µm.

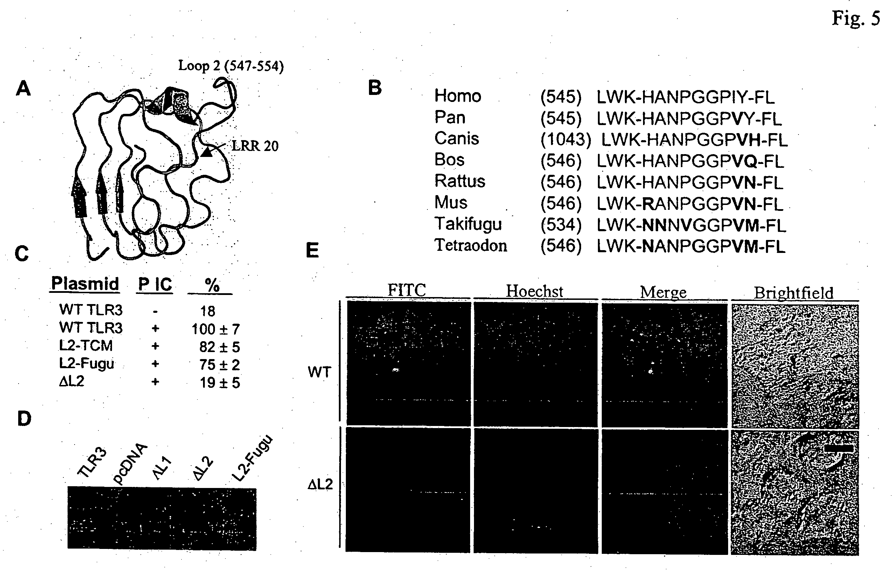

Figure 5 shows data in examination of the role of Loop2 on TLR3 function. A) A model of a section of TLR3 showing the location of Loop2. B) A comparison of the sequences in Loop2 from different species. The residues that are different from the human Loop2 sequence are shown in black. C) Activity assay of wild-type TLR3 and mutations in Loop2 of TLR3. D) Western blot analysis examining whether the mutations in Loop2 affect protein expression. E) In situ localization of the mutant deleted for Loop2 in comparison to wild-type TLR3. The presence of TLR3 in punctate spots, the nuclei, and merging of the two results are shown as identified above the micrographs. The bar in the lower micrograph represents 20 µm.

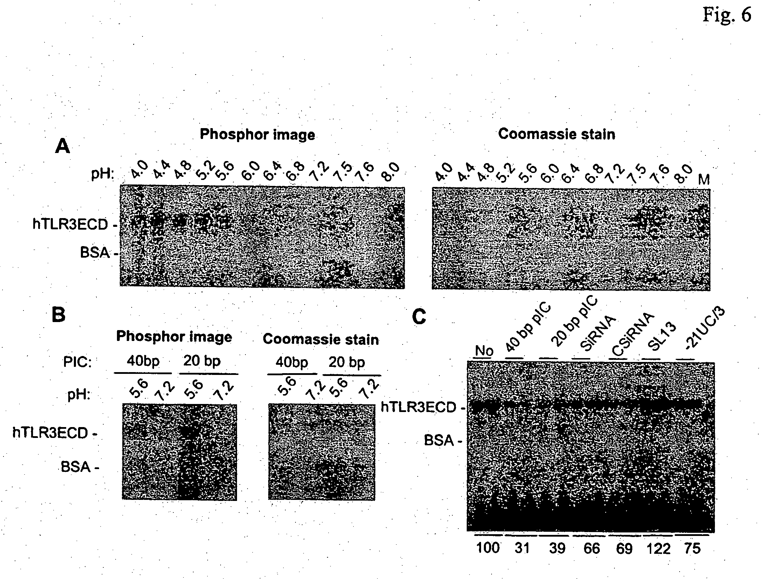

Figure 6 shows poly(I:C) binding by TLR3ECD in vitro. A) Crosslinking between TLR3 ECD and poly(I:C) as a function of pH. Poly(I:C) was radiolabeled by kinasing with ³²P-γ-ATP and T4 polynucleotide kinase. The crosslinking was performed with an equal mixture of TLR3ECD and BSA. The phosphorimage of the crosslinked products are shown in the image on the left and the Coomassie blue-stained gel is shown in the right image. B) Effect of poly(I:C) length on RNA crosslinking as a function of pH. Poly(I:C) of 40-bp and 20-bp were radiolabeled separately and used as probes. The phosphorimage and the Coomassie Blue-stained gel images are on the left and right, respectively. C) Competition for TLR3ECD binding to poly(I:C) by competitor RNAs added to the reactions at 2-4 fold of the 20-bp poly(I:C). The RNAs used are shown above the lanes in the gel image in which the RNA was added. The effect on crosslinking to the 20-bp poly(I:C) was quantified below the gel image.

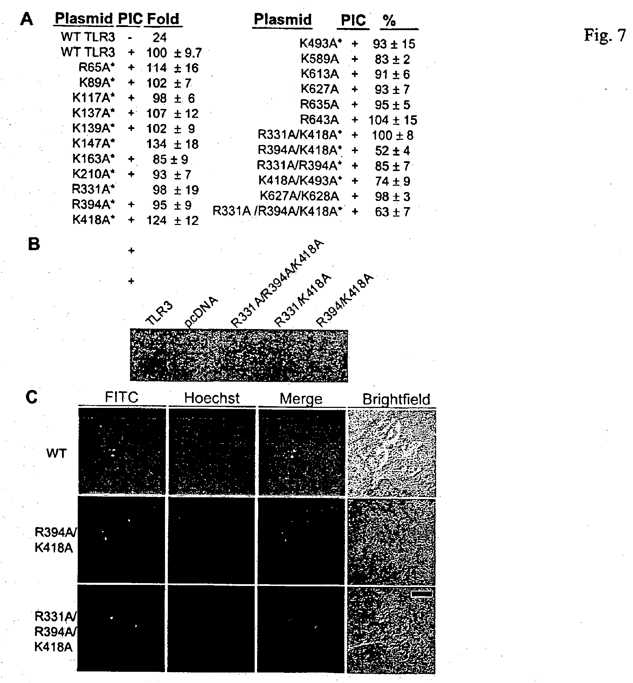

Figure 7 show data in examination of putative RNA binding residues in TLR3ECD. A) Summary of the effects of amino acid substitutions on TLR3 activity. The plasmids encoding wild-type or mutant TLR3 that were transfected into 293T cells are listed along with their effects on luciferase activity. Each value represents a minimum of six independent transfection assays. B) Western blot analysis of some of the mutant TLR3 proteins tested for activity in the cell-based reporter assay. C) Analysis of specific TLR3 mutants for intracellular locations in transfected 293T cells.

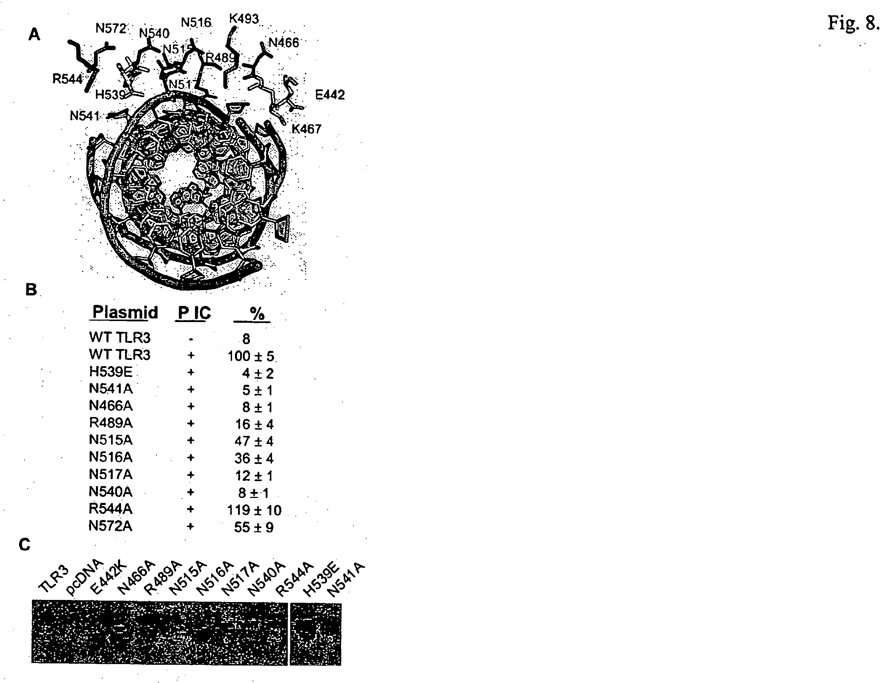

Figure 8 shows data in examination of the RNA-binding site in TLR3ECD reported by Bell et al. A) A model for poly(I:C) binding to TLR3. The residues that are proposed to be in close contact to poly(I:C) are shown. B) Summary of the effects of amino acid mutations in the charged surface in putative contact with poly(I:C). The plasmids encoding wild-type or mutant TLR3 that were transfected into 293T cells are listed along with their effects on luciferase activity. C) Western blot analysis of some of the mutants tested that had affects on TLR3 activity. The western was probed with the monoclonal antibody, IMG315A.

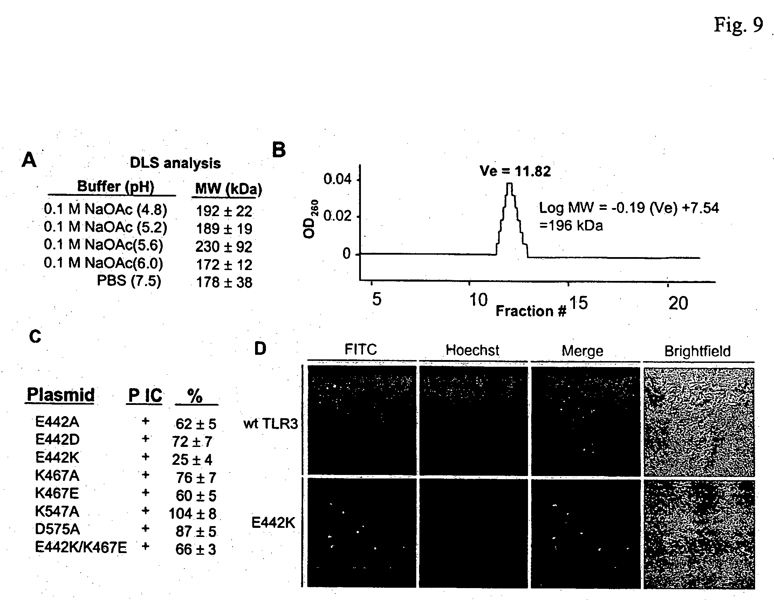

Figure 9 shows data in examination of residues that may mediate TLR3 oligomerization. A) Dynamic light scattering result of TLR3ECD examined as a function of pH. B) A gel filtration analysis of the elution profile of TLR3ECD. The fractions containing the peak of hTLRECD were detected by SDS-PAGE and staining the protein with silver. C) Effects of mutations in residues participating in TLR3 oligomerization on TLR3 activity. D) Intracellular localization of E442K, which is defective for TLR3 activity.

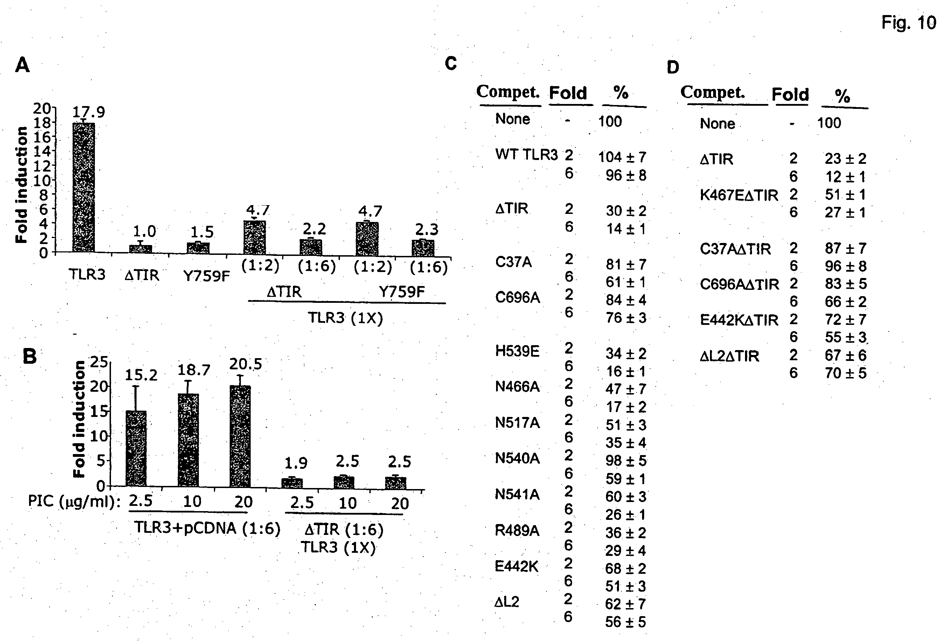

Figure 10 shows data in assessing the ability of TLR3 variants to act as dominant negatives. A) The ability of mutants ΔTIR and Y75F to activate TLR3 activity and to act as dominant negatives. In all of these assays, 1X denotes that the plasmid is present at 15 ng per transfection. The dominant negative assays were performed with 2X and 6X this concentration. B) A demonstration that increasing poly(I:C) induction could not reverse the dominant negative effect of ΔTIR. C) A summary of the assays for dominant negativity by several mutations that are defective for TLR3 activity. The mutants selected for analysis are also ones that are expressed well, as determined by Western blots. D) A summary of the results from selected mutants built into the context where the TIR domain was deleted (ΔTIR). This construction is useful to confirm the dominant negative result since the mutants cannot activate NF-kB in the absence of the TIR domain, thus reducing the background for the assay.

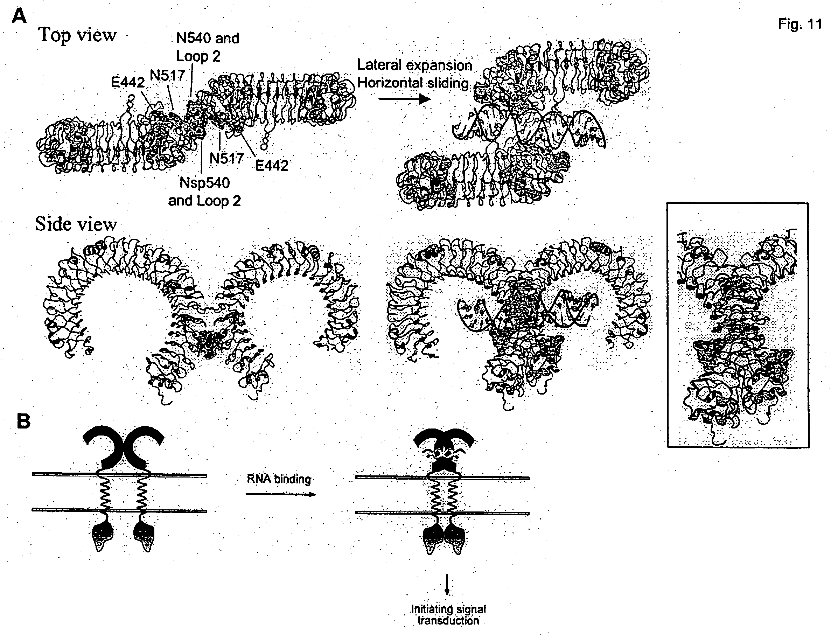

Figure 11 illustrates a model for the interaction between TLR3ECD subunits and with dsRNA. A) The top and side views of the 3ECD (PDB id 2A0Z) without and with a dsRNA (PDB id 1QC0). The ECD dimer as well as its complex with RNA was obtained through manual docking. The boxed panel highlights the interactions between the C-terminal portions of two ECD molecules in the RNA-bound state. However, the RNA was removed to allow better visualization of the proteins involved in this interaction. B) A cartoon model of full-length TLR3 illustrating how ECD ligand binding can lead to dimerization and subsequent activation of the TIR domain.

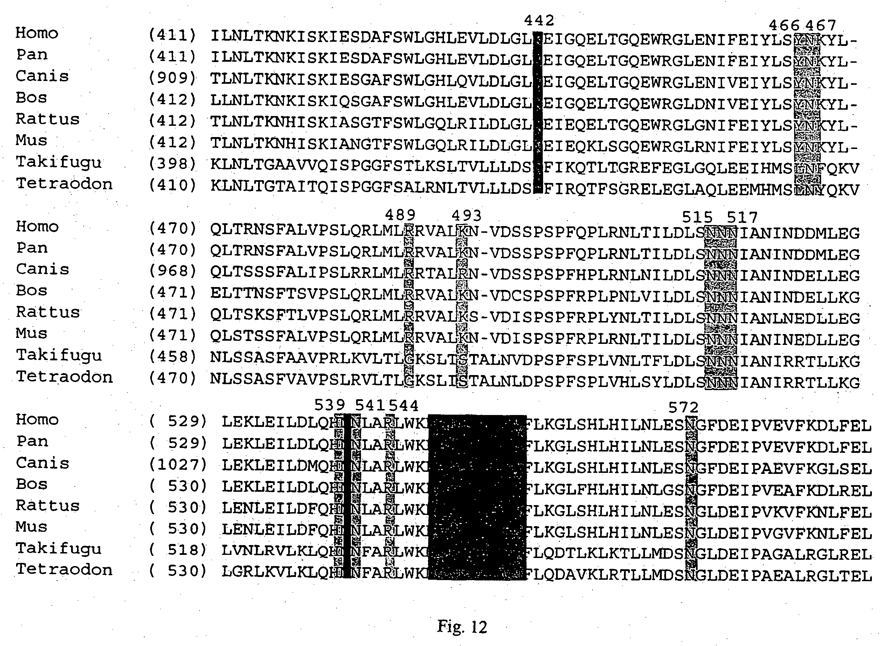

Figure 12 shows alignment of TLR3ECD homologous sequences across a broad range of vertebrates. The protein-protein interacting residues we identified are highlighted in red and RNA interaction residues in yellow.

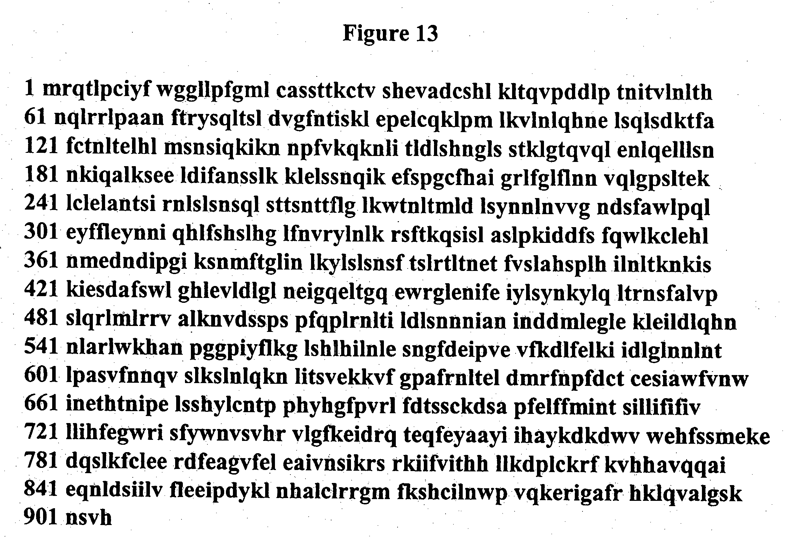

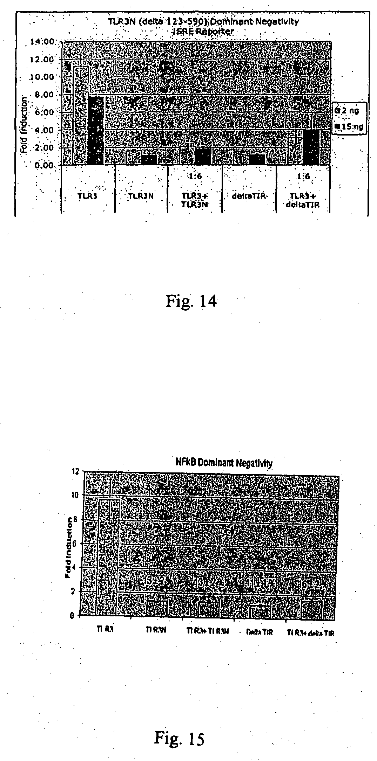

Figure 13 shows SEQ ID NO.:1 which is homo sapiens TLR3.

### DETAILED DESCRIPTION OF INVENTION

The invention relates to compositions and methods related to toll-like receptor (TLR) polypeptides. In some embodiments, the invention relates to managing TLR3 related diseases. In further embodiments, the invention relates to compositions for preventing and treating inflammation. In some embodiments, the invention relates to antagonists of TLR3, to amino acid sequences that act as dominant negative molecules, and to nucleic acid sequences that encode said amino acid sequences as defined in the claims. The invention relates to the manipulation of biological materials to evaluate TLR3 activity.

Toll-like receptors (TLRs) are type I transmembrane proteins that often recognize microbes once they have breached physical barriers such as the skin or intestinal tract mucosa, and activate immune cell responses. Thirteen TLRs (named simply TLR1 to TLR13) have been identified in humans and mice together, and equivalent forms of many of these have been found in other mammalian species.

A dominant negative mutation occurs when a mutant gene product affects the normal, wild-type gene product within the same cell. This usually occurs if the product of the mutation can still interact with the same elements as the wild-type product, but block some aspect of its function. The term "dominant negative inhibitor" and the like means a mutant gene product of a dominant negative mutation. As used herein, it is not intended to be limited in the manner in which the dominant negative inhibitor is made, and some embodiments contemplate that it is produced synthetically. It is also intended to include the mutant gene product that provides partial inhibition or function alteration, and it is not intended to require total inhibition.

The term "manage" when used in connection with a disease or condition means to provide beneficial effects to a patient being administered with a prophylactic or therapeutic agent, which does not result in a cure of the disease. A patient may be administered with one or more prophylactic or therapeutic agents to manage a disease so as to prevent the progression or worsening of the disease.

As used herein, the terms "prevent" and "preventing" include the prevention of the recurrence, spread or onset. It is not intended that the present invention be limited to complete prevention. In some embodiments, the onset is delayed, or the severity of the disease is reduced.

As used herein, the terms "treat" and "treating" are not limited to the case where the subject (e.g. patient) is cured and the disease is eradicated. Rather, the present description also contemplates treatment that merely reduces symptoms, and/or delays disease progression.

"Inflammation" or "inflammatory response" and the like means reaction of the body to injury or to infectious agent, allergic agent, abnormality in the regulation of the body's immune response to its own tissues, non-living foreign material, or a chemical irritation. The symptoms are redness, swelling, heat, and pain resulting from dilation of the blood vessels in the affected part with loss of plasma and leucocytes (white blood cells) into the tissues. Inflammation can be acute or chronic. Inflammatory responses include, but are not limited to, those attributable to tuberculosis, chronic cholecystitis, bronchiectasis, rheumatoid arthritis, Hashimoto's thyroiditis, inflammatory bowel disease (ulcerative colitis and Crohn's disease), silicosis and other pneumoconiosis, asthma, multiple sclerosis, hepatitis, chronic obstructive pulmonary disease, hay fever and other allergies, cardiovascular disease, implanted foreign body, systemic lupus erythematosus, and type 1 diabetes.

"Subject" means any animal, preferably a human patient, livestock, or domestic pet.

Some embodiments of the present invention provide mutant or variant forms of enzymes described herein. It is possible to modify the structure of a peptide having an activity of the enzymes described herein for such purposes as enhancing substrate specificity, stability, and the like. For example, a modified peptide can be produced in which the amino acid sequence has been altered, such as by amino acid substitution, deletion, or addition. For example, it is contemplated that an isolated replacement of a leucine with an isoleucine or valine, an aspartate with a glutamate, a threonine with a serine, or a similar replacement of an amino acid with a structurally related amino acid (*i*.*e*., conservative mutations) will, in some instances but not all, not have a major effect on the biological activity of the resulting molecule. Accordingly, some embodiments of the present invention provide variants of enzymes described herein containing conservative replacements. Conservative replacements are those that take place within a family of amino acids that are related in their side chains. Genetically encoded amino acids can be divided into four families: (1) acidic (aspartate, glutamate); (2) basic (lysine, arginine, histidine); (3) nonpolar (alanine, valine, leucine, isoleucine, proline, phenylalanine, methionine, tryptophan); and (4) uncharged polar (glycine, asparagine, glutamine, cysteine, serine, threonine, tyrosine). Phenylalanine, tryptophan, and tyrosine are sometimes classified jointly as aromatic amino acids. In similar fashion, the amino acid repertoire can be grouped as (1) acidic (aspartate, glutamate); (2) basic (lysine, arginine histidine), (3) aliphatic (glycine, alanine, valine, leucine, isoleucine, serine, threonine), with serine and threonine optionally be grouped separately as aliphatic-hydroxyl; (4) aromatic (phenylalanine, tyrosine, tryptophan); (5) amide (asparagine, glutamine); and (6) sulfur -containing (cysteine and methionine) (*See e.g.,* Stryer (ed.), Biochemistry, 2nd ed, WH Freeman and Co. [1981]). Whether a change in the amino acid sequence of a peptide results in a functional homolog can be readily determined by assessing the ability of the variant peptide to produce a response in a fashion similar to the wild-type protein using the assays described herein. Peptides in which more than one replacement has taken place can readily be tested in the same manner.

The term "antibody", as used herein, refers to a molecule specifically binding to an antigen, and includes dimeric, trimeric and multimeric antibodies, and recombinant, processed and humanized antibodies. Also, an antibody may be a whole antibody or a functional fragment of an antibody molecule. The term "functional fragment of an antibody molecule" indicates a fragment retaining at least its antigen binding function, and include Fab, F(ab'), F(ab')₂, scFv, dsFv, and diabody. Techniques for the preparation and use of the various antibodies are well known in the art. For example, antibody fragments may be obtained using proteolytic enzymes (e.g., a whole antibody is digested with papain to produce Fab fragments, and pepsin treatment results in the production of F(ab')2 fragments), and may be preferably prepared by recombinant DNA techniques. An isolated antibody any collected composition containing the antibody. Preferably the concentration of said antibody is greater than that found in blood serum.

As used herein, "humanized" forms of non-human (e.g., murine) antibodies are chimeric antibodies that contain minimal sequence, or no sequence, derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, Fv framework region (FR) residues of the human immunoglobulin are replaced by corresponding non-human residues. Furthermore, humanized antibodies may comprise residues that are not found in the recipient antibody or in the donor antibody. These modifications are generally made to further refine antibody performance. In general, the humanized antibody will comprise substantially all of at least one, and typically two, variable domains, in which all or substantially all of the hypervariable loops correspond to those of a nonhuman immunoglobulin and all or substantially all of the FR residues are those of a human immunoglobulin sequence. The humanized antibody may also comprise at least a portion of an immunoglobulin constant region (Fc), typically that of a human immunoglobulin. Examples of methods used to generate humanized antibodies are described in U.S. Pat. No. 5,225,539 to Winter et al..

Importantly, early methods for humanizing antibodies often resulted in antibodies with lower affinity than the non-human antibody starting material. More recent approaches to humanizing antibodies address this problem by making changes to the CDRs. See U.S. 20040162413. In some examples, the present description provides an optimized heteromeric variable region (e.g. that may or may not be part of a full antibody other molecule) having equal or higher antigen binding affinity than a donor heteromeric variable region, wherein the donor heteromeric variable region comprises three light chain donor CDRs, and wherein the optimized heteromeric variable region comprises: a) a light chain altered variable region comprising; i) four unvaried human germline light chain framework regions, and ii) three light chain altered variable region CDRs, wherein at least one of the three light chain altered variable region CDRs is a light chain donor CDR variant, and wherein the light chain donor CDR variant comprises a different amino acid at only one, two, three or four positions compared to one of the three light chain donor CDRs (e.g. the at least one light chain donor CDR variant is identical to one of the light chain donor CDRs except for one, two, three or four amino acid differences).

In some examples, the description relates to amino acid residues in human TLR3 found to have bioactivity. Specifically, we discovered that mutations of residues 547-554 abolished TLR3 bioactivity as measured by NF-κβ activation. Moreover, co-transfection of competent human Hek293 cells with bioactive and mutant human TLR3 genes resulted in loss of TLR3 activity, demonstrating the ability of the mutant TLR3 molecule to act as a dominant negative molecule. The antagonistic activity of the TLR3 mutant molecules provide a unique tool to interfere with TLR3 activity, which proves beneficial in preventing and treating TLR3-mediated diseases including inflammation. We have also identified that a mutant TLR3 interacts with other TLRs including TLR9.

TLR3 activation by ligands derived from viral RNA, bacterial RNA or endogenous necrotic cells results in a signaling cascade that ultimately leads to NF-κβ activation and downstream secretion of pro-inflammatory cytokines and chemokines including IL-6, RANTES, TNF-α, MCP-1 to cite a few. It is well established from experiments performed in animal models that inflammatory cytokines and chemokines play a critical role in initiating and maintaining the local inflammatory response and subsequent tissue destruction. Therefore antagonist TLR3 agents such as dominant negative TLR3 molecules is beneficial in preventing or treating inflammatory conditions.

Activation of TLR3 in the lungs has been suggested to mediate exacerbation of the local inflammatory response. Thus, the use of dominant negative TLR3 molecules described herein can prove beneficial for the treatment or prevention of pulmonary diseases including asthma, asthma exacerbation, microbial-associated pneumonia, sarcoidosis and cystic fibrosis.

The presence of necrotic cells in the synovial fluid of rheumatoid arthritis patients has been shown to activate TLR3 and downstream secretion of inflammatory mediators, suggesting a role for TLR3 activation in modulating disease outcome in rheumatoid arthritis patients. Therefore, the dominant negative TLR3 molecules are beneficial for the treatment of autoimmune diseases including RA, psoriatic arthritis, and giant cell arteritis.

TLR3 activation in the liver is an important event in mediating liver damage. Thus the use of TLR3 antagonist molecule such as dominant negative TLR3 molecules may alleviate liver damage and may be used for the prevention of treatment of liver damage including liver fibrosis.

The dominant negative TLR3 molecule may be used in treating autoimmune diseases such as systemic lupus erythematosus and lupus nephritis given the association between TLR3 activation in the kidney and disease activity in animal models of lupus nephritis.

The TLR3 dominant negative molecule may be used for the treatment of fibrotic-associated diseases based on recent findings showing cells derived from fibrosis-proned animals were highly susceptible to TLR3 ligands as measured by enhanced production of inflammatory cytokines such as TNF-α.

In some examples, the description relates to the use of DNA encoding for dominant negative of human TLR3 molecule described herein in combination with standard therapies including antimicrobial agents, corticosteroids and immuno-modulatory agents is also claimed for the treatment or prevention of diseases described herein.

The invention relates to the use of polypeptide molecules described herein for the treatment or prevention of the diseases described above.

The invention relates to administration of the dominant negative molecules by using subcutaneous, oral, intravenous intradermal or intranasal routes for the treatment of diseases described above.

### TOLL-LIKE RECEPTOR 3 (TLR3) MUTANTS AND SEQUENCES

The structure of the human TLR3 ectodomain (ECD) was solved by X-ray crystallography, leading to a number of models concerning TLR3 function. (Choe, J., Kelker, M. S., and Wilson, I. A. (2005). Science 309, 581-585 and Bell, J. K., Botos, I., Hall, P. R., Askins, J., Shiloach, J., Segal, D. M., and Davies, D. R. (2005) Proc Natl Acad Sci USA 102, 10976-10980). The structure revealed four pairs of cysteines that are putatively involved in disulfide bond formation. There are two loops that protrude from the central solenoid structure of the protein. We examined the recombinant TLR3 ECD
for disulfide bond formation, poly(I:C) binding, and protein-protein interaction. We also made over 80 mutations in the residues that could affect these features in the full-length TLR3 and they were examined for effects in TLR3-mediated NF-κB activation. A number of mutations that affected TLR3 activity also affected the ability to act as dominant negative inhibitors of wild-type TLR3. Loss of putative RNA binding did not necessarily affect dominant negative activity. All of the results support a model where a dimer of TLR3 is the form that binds RNA and activates signal transduction.

The recognition of foreign molecules by the innate immune receptors can lead to the activation of a signaling cascade, changes in gene expression, and production of cytokines by effector cells. The consequence of this pathway dictates the outcome of an immune response through modulation of T- and B-lymphocyte activation in the adaptive immune pathways.

At least eleven TLRs have been identified in the human genome. TLR3 recognizes poly(I:C), a synthetic double-stranded (ds) RNA analog, as well as viral dsRNA, presumably formed during viral infection. A TLR3 knockout mice is unable to mount a full response to cytomegalovirus infection and decreased the cytotoxic T cell response after the initial infection in mice. These results support a role for TLR3 in modulating the host immune response against microbial challenge.

Upon ligand binding, TLR3 can, through adaptor proteins, activate the transcription factor NF-κB, which translocates to the nucleus to modulate gene expression. The site of action for TLR3 activation is likely in or near intracellular vesicles, although some cell-surface expression is observed in human embryonic kidney cells.

The structures of the TLR3 ectodomain have been elucidated by X-ray crystallography by two groups, leading to several predictions about how the structure affects function. The TLR3 ECD is shaped as a solenoid horseshoe, characteristic of proteins with multiple leucine-rich repeats (LRRs). A number of features in the protein structure could impact TLR3 function. Based on surface charge properties and the location of glycosylations, the region proposed to bind dsRNA was proposed to be free of glycosylation. The structures from the crystal packing suggest that the C-terminal portions of two subunits interact through ionic interactions. TLR3 ECD is also predicted to contain four disulfide bonds near the N- and C-termini of the solenoid that may stabilize the ECD structure. Lastly, there are two protruding loops in the 3ECD solenoid that may contribute to TLR3 function.

Analysis of how the structural features of the 3ECD impact function is an active area of research. All of the predicted N-linked glycosylation sites in TLR3 ECD have been mutated and two have been shown to be important for TLR3 activity in transfected HEK 293T cells. Several of the cysteines putatively involved in disulfide bond formation have been mutated. Bell et al., (2005) Proc Natl Acad Sci USA 102, 10976-10980, examined the effects of many mutations throughout the 3ECD of TLR3 and demonstrated that two residues, H539E and N541A are affected for activity in cultured 293T cells and prevented recombinant 3ECD from binding to dsRNA in a gel-filtration based assay. A thorough mutational analysis of residues neighboring H539E and N541A had more modest effects on TLR3 activity.

We have made over eighty mutants in TLR3 that are predicted to affect disulfide bond formation, dimerization, and RNA binding and examined their effects in a cell-based assay for TLR3 activation of downstream reporter expression. Biochemical assays for the properties of the TLR3 ECD produced in human cells are also examined. The effects of select mutants that decreased TLR3 activity were examined for effects on protein expression, cellular localization, and for the ability to act as dominant negatives of co-transfected wild-type copy of TLR3.

NF-kB activation assay provided for TLR3 function. Human embryonic kidney (HEK) 293T cells were used to analyze how mutations in TLR3 will affect TLR3 function and localization. 293T cells are useful for this assay since they do not express detectable levels of endogenous TLR3 (Fig. 1A, box). Briefly, cells cultured in 96-well plates to ∼80% confluence were transfected with a mixture of three plasmids: one to express either wild-type or mutant TLR3, a second to express the firefly luciferase driven from promoter containing NF-kB binding sites, and a third to express the Renilla luciferase from the Herpesvirus thymidine kinase promoter. The Renilla luciferase serves as a transfection control. Poly(I:C) purchased from Invivogen was used as the ligand to induce TLR3-mediated NF-κB activation.

Our assay can respond to up to 75 ng of the TLR3 plasmid in the transfection (Fig. 1A), but our standard assay uses 15 ng of plasmid per transfection to ensure that the signal will not be saturated. The ratio of firefly luciferase to Renilla luciferase activity was calculated for all the samples induced with poly(I:C) as well as with the buffer alone control. The fold of poly(I:C) induced TLR3 activation was the normalized to the wild-type control (100%) assayed in the same experiment. A minimum of four-fold induction of TLR3 activity by the addition of poly(I:C) was detected in all of our assays (Fig. 1B). As an example, a mutant TLR3 can reduce activity to background (Fig. 1B).

Cysteines are involved in disulfide bond formation. Cysteines that putatively form disulfide bonds to cap the ends of the ECD are: C28 and C37, C95 and C122, C649 and C677, and C651 and C696 (Fig. 2A; 1, 2). The cysteines are conserved in all species in which the TLR3 orthologs have been identified, suggesting that the disulfide bonds they form are important for TLR3 function (Fig. 2B).

We first attempted to determine whether the formation of the disulfides could be detected in tryptically digested fragments of TLR3ECD using mass spectrometry (Fig. 2C). Reduced cysteines are acetylated by iodoacetamide and the ones involved in disulfide bond formation are not. The MS spectra obtained in the non-reduced tryptic digest resulted in a signal at 1526.81 m/z, which corresponds to the modified peptide CTVSHEVADCSHLK if a disulfide was originally present (Fig. 2C top panel). To confirm the sequence and structural assignment of this peptide, tandem MS was performed (Fig. 2D). Nearly the entire C-terminal y ion series (y2 to y9) was observed, confirming the peptide sequence assignment. More significant is the observation of the b series ions b10 - b13, were all 2 Da lower than what would be expected for the reduced peptide. These results confirm the presence of a disulfide bond between Cys28 and Cys37. The other three pairs of disulfides were not observed despite repeated attempts, suggesting that they may either be present at lower abundances or that the peptides containing these disulfides could not be ionized under the conditions used.

To assess the functional relevance of the disulfide bonds, we mutated each participating cysteine to alanine. Mutants C28A, C37A, C95A, C122A, C649A, C651A, and C696A all resulted in TLR3 activities near background (Fig. 3A). We also examined whether replacements of some of the cysteines with serine or methionines would affect activity. All of the changes in residues C37, C95, C122, C649, C651, and C696 resulted in activities at or near the background level. Therefore, the cysteines involved in disulfide bond formation are important for TLR3 function. In contrast, mutations of cysteine residues that are not predicted to participate in disulfide bond formation (C242, or C356, or both) had only minimal effects on TLR3 activity (Fig.3A).

Mutations in the disulfide-forming cysteines could affect several properties of TLR3, including its expression, stability, and/or intracellular localization. To examine whether TLR3 expression was affected, lysates from transfected cells were subjected to western blots with a TLR3-specific monoclonal antibody. All cysteine mutants were expressed at levels comparable to WT (Fig. 3B). To examine whether the mutant proteins are affected in the intracellular localization of TLR3, we immunostained transfected HEK 293T cells for TLR3 and confirmed that TLR3 localizes to intracellular acidic organelles in a punctate distribution. The spots co-localized with acidic vesicles that can be stained with LysoTracker. Mutant C242A, which is not implicated in disulfide bond formation and suffered no significant loss of activity in the cell-based assay, has an appearance similar to wt TLR3 (Fig. 3C). Among the mutants involved in disulfide bond formation, C651A and C696A had suffered no obvious changes to wild-type TLR3 localization while C37A and C122A had more diffused signals rather than discrete spots (Fig. 3C). A brighter signal was also seen throughout the cytoplasm, suggesting some loss of localization to intracellular vesicles.

We also examined the cell surface distribution for mutants C37A and C122A by fluorescence activated cell sorting. C37A and C122A are reduced for cell surface expression (Fig. 3D). This data suggests that cysteines involved in disulfide bonds formation in TLR3 are important for activity and the mutants are expressed at levels comparable to WT, but some are affected in their intracellular localization. This change in localization contributes to the loss of TLR3 activity.

Structures that project out from the central solenoid structure of TLR3 ECD could provide features important for TLR3 function. A loop within the LRRs of TLR9 has been hypothesized to interact with the ligand, CpG DNA. Bell, et al., (2003). Trends Immunol. 24, 528-533. TLR3 has two loops in the ECD solenoid. The first, called Loop1, resides in LRR12 (residues 335-343) and is rich in serine residues (Fig. 4A and B). An examination of the sequence of Loop1 revealed that it is variable in its sequence and length. For example, while the mammalian Loop1 is composed of eight residues, the equivalents from fish have only six residues (Fig. 4B).

We first replaced the central six residues of Loop1 (SISLAS) with the six-residue sequence: CCPGCC that could bind the FlAsH dye. See e.g, Griffin et al., (1998). Science 281, 269-272. Our intention was to fluorescently label TLR3 by its binding to the FlAsH dye. However, this construct, LI-TCM, did not bind the FlAsH dye well, perhaps due to steric constraints. Nonetheless, the construct was as active as the WT for NF-κB reporter activity (Fig.4C). Next, we changed four of the residues in TLR3 Loop1 from QSISLASL to QSTALTSH in a construct named L1-4M. Again, more than 85% of the TLR3 activity was retained. Lastly, we deleted Loop1 altogether (ΔL1) and found the resultant construct to retain greater than 80% of the wild-type TLR3 activity (Fig.4C). Western analysis showed that the proteins were made similar to WT (Fig. 4D). In localization experiments, ΔL1 formed intracellular specks in a manner indistinguishable from WT (Fig. 4E). These results demonstrate that Loop1 is not essential for TLR3 function.

The second loop in TLR3 ECD resides within LRR20 (residues 547 to 554) (Fig. 5A). Unlike Loop1, several residues are highly conserved (Fig. 5B). When the tetracysteine motif was inserted into the apex of Loop2, construct L2-TCM resulted in a protein that retained 82% of the activity of the WT (Fig. 5C). Replacement of the Loop2 sequence in TLR3 with the comparable sequence from Takifugu in construct L2-Fugu retained 75% of the wild-type function, confirming that there is some flexibility of the Loop2 sequence. However, a deletion of Loop2 in construct ΔL2 resulted in activity near background (Fig. 5C). ΔL2 protein was expressed at wild-type levels, when detected in western blots, suggesting that protein expression is not responsible for the defect (Fig. 5D). Furthermore, ΔL2 is apparently expressed as punctate spots similar to WT, indicating that different intracellular localization is not a cause of the defect (Fig. 5E).

Poly(I:C) binds the TLR3 ECD. TLR3 responds to dsRNA that could be generated during viral inflection. dsRNA binding could occur either directly or through an accessory protein, such as CD14. Assays for dsRNA binding by the TLR3 ECD are limited. Choe et al., (2005) Science 309, 581-585, demonstrated an electrophoretic mobility shift of TLR3 ECD upon poly(I:C) binding, while Bell et al. observed a complex between the TLR3 ECD and RNA in a gel-filtration assay. We used a UV crosslinking assay to examine TLR3ECD interaction with poly(I:C) radiolabeled at the 5' terminus. Since TLR3 is localized to acidic vesicles, we also assessed whether the pH of the reaction would affect TLR3 interaction with poly(I:C). BSA was added to TLR3ECD at an equal molar ratio to provide an internal control. The TLR3ECD was crosslinked to poly(I:C) while BSA was not. We note that the poly(I:C) used in these assays are capable of inducing TLR3 activation of NF-κβ activity. Furthermore, crosslinking to poly(I:C) was most effective at acidic pH (Fig. 6A).

Since commercial preparations of poly(I:C) is heterogeneous in mass, we prepared poly(I:C) of 40 and 20 bp for the crosslinking assay. Both were crosslinked to TLR3ECD (Fig. 6B). Lastly, to determine whether TLR3ECD specifically recognized poly(I:C), we examined whether crosslinking to the radiolabeled 20-bp poly(I:C) could be completed with other potential ligands. Competing ligands used were unlabeled poly(I:C) of 20- or 40-bp, two siRNAs of 21 bp, a highly structured RNA of 13-nt (Kim et al., 2000, Nat Struct Biol. 7, 415-423), and a 33-nt single-stranded unmodified RNA named -21/13 (Siegel et al., (1997). Proc Natl Acad Sci USA 94,11238-11243.). The two preparations of poly(I:C) were effective competitors when present at 2-4 fold above the labeled ligand, reducing the radiolabeled complex to less than 40% of the reaction lacking a competitor (Fig. 6C). The two siRNAs, were weaker competitors, reducing poly(I:C) crosslinking to approximately a third. The structured and single-stranded RNA were the worst competitors. These results provide biochemical evidence that hTLR3ECD could specifically recognize poly(I:C) in the absence of accessory proteins.

Several residues in the TLR3 ECD were proposed to contact dsRNA. We made alanine mutants of most of the predicted residues as well as the basic residues near the Loop2 of TLR3 ECD. All of the single amino acid changes were largely unaffected in TLR3 activity (Fig. 7A). Combinations of two and three mutations in TLR3 did have some effect, but TLR3 activity remained at more than half of the level for WT. Western blots showed that several of the single and multiple mutants had expression levels and in situ localization similar to that of WT (Fig. 7B and C). Furthermore, none of the individual residues could be assigned as being critical for TLR3 activity.

The RNA-binding surface of TLR3 may be an asparagine-rich surface on the side and C-terminal third of the 3ECD solenoid. Mutations of two residues, H539 and N541, had severe effects on TLR3 activity in cell-based assays. To better visualize the potential contact sites, we used the coordinates of a structure of poly(I:C) (PDB ID code 1QC0) and attempted to dock the molecule into this portion of the TLR3 ECD (Fig. 8A). Residues R544, N540, N516, and N466 are all within the patch that could interact with poly(I:C). Interestingly, when viewed from the end of the poly(I:C) helix, residues E442 and K467 predicted to be involved in TLR3 oligomerization, are at the other side of the RNA molecule and could either contact poly(I:C) and/or be affected by poly(I:C) binding.

We made mutants H539E and N541A and other mutations in the putative RNA-binding surface. Mutants H539E and N541A had TLR3 activity near background levels (Fig. 8B). Furthermore, adjacent mutations not previously tested by Bell et al., N466A and N540A, also reduced TLR3 activity to background level.

Some changes at the same positions in TLR3 that we tested had different effects than reported, possibly due to the identity of the altered residue. It was reported that N515D and N516L did not affect TLR3 activity. We found that N515A and N516A reduced TLR3 activity to 47 and to 36% of WT. Also, mutant N572A had 55% activity of the WT. Lastly, R489A and N517A reduced TLR3 activity to nearly background while Bell et al., reported that R489A and N517A had more than 50% activity. Western blots of these mutant proteins showed that they are produced in 293T cells, although some, such as N515A, were present in slightly lower amounts compared to WT and could have contributed to decreased TLR3 activity (Fig. 8C). The results demonstrate that charged surface characterized by enriched asparagines in LRR17 to 20 on the side of the solenoid is important for TLR3 function.

The putative RNA-binding patch is spatially close to the putative dimerization domain in TLR3 ECD, suggesting a relationship between these two activities. The TLR ECD could exist as a dimer in both 3-D and 2-D crystal lattices. However, that observation might be due to the high protein concentrations needed for crystal formation. Therefore, we examined whether hTLR3ECD could exist in an oligomeric state at lower protein concentrations using dynamic light scattering analysis. The mass of hTLR3ECD monomer is ∼100 kD, as determined by mass spectrometry and SDS-PAGE. In a PBS solution, hTLR3ECD (at 25 µg/ml) had a hydrodynamic radius of corresponding to a protein of 178 ± 36 kDa (Fig. 9A). When tested in sodium acetate buffered from pH 6.0 to 4.8, the mass of hTLR3ECD in solution was between 172 kDa to 230 kDa, demonstrating that hTLR3ECD can exist as a dimer in solution in the absence of ligand, and at pHs typically found in an acidic vesicle. We also subjected hTLR3ECD to gel filtration chromatography in comparison to molecular mass markers and it eluted with a peak at 196 kDa, confirming that hTLR3ECD exists predominantly as a dimer (Fig. 9B).

Residues E442 and K467, and also K547 and D575 were predicted to form salt bridges as a part of the interaction between TLR3 subunits. A number of amino acid substitutions were made to test this prediction. Mutant K467A and K467E, only reduced TLR3 activity to 76 and 60% of WT, respectively (Fig. 9C). Mutants E442A, E442D retained more than 62% of the wild-type activity, but changing E442 to a lysine reduced TLR3 activity to 25% of the WT. Localization of E442K in 293T cells showed that it is expressed similar to WT (Fig. 9D). Also, K547A and D575A mutants had negligible effect on activity of the protein as measured by NF-κB activation (Fig. 9C). However, we note that a double mutant E442K/K467E had 66% of the wild-type TLR3 activity. This suggests that the reduction of activity seen with E442K can be partially compensated with the K467E mutation. These results identify that a negatively charged residue at position 442 is important for TLR3 function, but our results do not support the idea that these residues form simple salt bridges since neither E442A nor K467A reduced TLR3 activity significantly. It is possible that that some changes at this position could be better compensated by a network of interactions involving two ECD molecules.

Given the difficulty to assessing the oligomerization state of TLR3 in cells, we used a genetic assay to assess whether mutant versions of TLR3 could suppress the activity of wild-type TLR3, i.e. to act as a dominant negative. The mechanistic basis for the dominant negative activity of a mutant TLR3 is not understood, and even though the inventor does not intend that the claims be limited by any particular mechanism, two likely possibilities exist: 1) a mutant TLR3 is unaffected for binding to the wild-type TLR3, but cannot carry out other activities needed to activate gene expression. Therefore, the mutant protein traps the WT in an inactive state. 2) The mutant protein exists as monomer and retains the ability to titrate ligands and/or accessory factors away from the WT.

The TLR family proteins consist of an extracellular leucine-rich repeat (LRR), a transmembrane region (TM) and a cytoplasmic tail containing a Toll/IL-1 receptor homology (TIR) domain. To examine the basis for dominant negativity/dimer formation, we used ΔTIR, a known dominant negative version of TLR3 that lacks the TIR domain. See Funami et al., (2004). Int. Immunol. 16, 1143-1154. Since ΔTIR is inactive for TLR3 activity, all of the output of the assay is from the co-transfected wild-type TLR3. At 2- or 6-fold molar excess of the wild-type TLR3, ΔTIR suppressed TLR3 activity to 26 and 12%, compared to an assay containing wild-type TLR3 challenged with comparable amounts of the empty vector (Fig. 10A). If the dominant negative effect of ΔTIR occurs by titrating away the ligand poly(I:C), then increasing poly(I:C) concentration should at least partially reverse the dominant negative effect. To test this, a four- or eight-fold higher concentration of poly(I:C) were added to the cells and no significant change in the dominant negative effect of ΔTIR was observed (Fig. 10B), suggesting that dominant negative effect is not due to ΔTIR titrating away the ligand. Similar results were obtained even when ΔTIR was present at 1:1 ratio to that of WT.

If the dominant negative activity were due to protein-protein interaction, then mutations that affect RNA-binding without affecting protein-protein interaction would be dominant negative. We tested mutants H539E and N466A. Both mutants retained their ability to act as dominant negatives, reducing TLR3 activity to 16 and 17% respectively when present at six molar excess of WT, comparable to the effects of ΔTIR (14%) (Fig. 10C).

We also found that mutant Y759F, a mutation that abolishes TIR function was also a dominant negative to similar levels as ΔTIR (Fig. 10A). Together with the results from ΔTIR, two properties are required for TLR3 activity: proper interaction between ECDs and between the TIRs.

The dominant negative assay was used to assess whether various mutations in TLR3 that significantly reduced TLR3 activity can retain protein-protein interaction. Cysteine mutants C37A and C696A were only able to reduce wild-type TLR3 activity to 61 and 76%, respectively, at six molar excess of WT (Fig. 10C). When constructed as a version lacking the TIR domain, C37A and C696A both were poor dominant negatives (Fig. 10D) confirming that the disulfide-forming cysteines are required for proper protein-protein interaction. The disulfides may be contributing to protein-protein interaction indirectly, by affecting the stability of the protein and/or proper localization of TLR3.

Mutant E442K that mapped to be at the right edge of the poly(I:C) binding surface Fig. (8A) and predicted to act in dimerization, was also a poor dominant negative. At six molar excess to the WT, E442K could only reduced wild-type TLR3 activity to 51% (Fig. 10C). ΔTIR version of E442K named E442KΔTIR was also a poor dominant negative (Fig. 10D), supporting the hypothesis that E442 contributes to protein-protein interaction.

Mutant K467E was hypothesized to play a role in 3ECD dimerization. However, since this mutant retained 60% of the TLR3 activity, we tested it for dominant negativity only when it lacked the ΔTIR domain. K467EΔTIR inhibited TLR3 activity to 27% at six fold molar excess, suggesting that mutation K457E did not affect dominant negativity (Fig. 10D).

Another mutant that affected TLR3 activity dramatically was ΔL2. Both ΔL2 and ΔL2ΔTIR were poor dominant negatives (Fig. 10C, 10D). Based on these results, we propose that E442 and Loop2, that are near the poly(I:C) binding surface, are required for interactions between TLR3 subunits.

Mutants N517A, N540A, and N541A had different abilities to inhibit the activity of WT TLR3, ranging from N517A and N541A that were able to partially retain dominantly negative activity to N540 that is a poor dominant negative. These results suggests some of the residues in the asparagine-rich surface of the 3ECD that putatively contacts poly(I:C) can participate in protein-protein interaction to result in a dominant negative phenotype.

We find that the recombinant TLR3ECD protein can be demonstrated to contain at least one disulfide bond that involves C28 and C37, as determined by mass spectrometry analysis. Furthermore, all of the cysteines putatively involved in disulfide bond formation are involved in TLR3 activity. We have also demonstrated that Loop1 within LRR12 of the 3ECD is dispensable for TLR3 activity. In fact, Loop1 may be useful as a place to insert a specific tag to follow TLR3 localization. We also demonstrated that hTLR3ECD can be crosslinked to poly(I:C) in pH conditions similar to that of acidic vesicles and that non-dsRNA are poor competitors for this crosslinking between TLR3 and poly(I:C). Also, TLR3ECD appears to exist as a dimer in solution in the absence of ligand.

There is an overlap in the TLR3 ECD residues that are required for poly(I:C) binding with those required for dominant negativity, the mechanistic basis of which is likely due to the interaction between a nonfunctional protein binding to a wild-type TLR3 through their ECD domains. Using the dominant negative assay, mutations that severely affected TLR3 activation of downstream reporter activity can be separated into those that retain the ability to act as dominant negatives and those that cannot. Interestingly, some, but not all of the putative RNA-binding surface in TLR3 are beneficial for dominant negative effect of TLR3. Also, Loop2 in LRR20 is beneficial for dominant negativity, suggesting a role in protein-protein interaction. Our mutational analysis supports claims for the interactions between 3ECD subunits. Most of the residues, including H539 and N541 that are suggested to bind RNA have a considerable overlap between the activities of RNA binding and dimerization.

We believe that TLR3 can exist in an oligomerized state in the absence of ligand mostly through Loop2 interactions. However, the ligand binding will cause rearrangement in the dimer leading to sliding of the two molecules towards each other laterally while the two molecules are being pushed to accommodate the dsRNA (Fig. 11A). In this ligand bound form, then residues E442 and N517 will interact primarily with the dsRNA to stabilize the complex. The resultant conformational change due to the sliding of the protein subunits may stimulate the interaction of the TIR domains, the subsequent dimerization of which, will lead to the activation of the signal transduction pathway (Fig. 11B).

### PHARMACEUTICAL COMPOSITIONS

The compositions comprising the active compound include bulk-drug compositions useful in the manufacture of pharmaceutical compositions (e.g., impure or non-sterile compositions) and pharmaceutical compositions (i.e., compositions that are suitable for administration to a patient) that can be used in the preparation of unit dosage forms. Such compositions optionally comprise a prophylactically or therapeutically effective amount of a prophylactic and/or therapeutic agent disclosed herein or a combination of those agents and a pharmaceutically acceptable carrier. Preferably, compositions of the invention comprise a prophylactically or therapeutically effective amount of the active compound and another therapeutic or prophylactic agent, and a pharmaceutically acceptable carrier.

The term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the active compound is administered. Such pharmaceutical vehicles can be liquids, such as water and oils, including those of petroleum, animal, vegetable or synthetic origin, such as peanut oil, soybean oil, mineral oil, sesame oil and the like. The pharmaceutical vehicles can be saline, gum acacia, gelatin, starch paste, talc, keratin, colloidal silica, urea, and the like. In addition, auxiliary, stabilizing, thickening, lubricating and coloring agents can be used. When administered to a patient, the pharmaceutically acceptable vehicles are preferably sterile. Water can be the vehicle when the active compound is administered intravenously. Saline solutions and aqueous dextrose and glycerol solutions can also be employed as liquid vehicles, particularly for injectable solutions. Suitable pharmaceutical vehicles also include excipients such as starch, glucose, lactose, sucrose, gelatin, malt, rice, flour, chalk, silica gel, sodium stearate, glycerol monostearate, talc, sodium chloride, dried skim milk, glycerol, propylethyleneglycol, water, ethanol and the like. The present compositions, if desired, can also contain minor amounts of wetting or emulsifying agents, or pH buffering agents.

The present compositions can take the form of solutions, suspensions, emulsion, tablets, pills, pellets, capsules, capsules containing liquids, powders, sustained-release formulations, suppositories, emulsions, aerosols, sprays, suspensions, or any other form suitable for use. In one embodiment, the pharmaceutically acceptable vehicle is a capsule (see e.g., U.S. Pat. No. 5,698,155).

The active compound and optionally another therapeutic or prophylactic agent may be formulated in accordance with routine procedures as pharmaceutical compositions adapted for intravenous administration to human beings.
Typically, the active compound(s) for intravenous administration are solutions in sterile isotonic aqueous buffer. Where necessary, the compositions can also include a solubilizing agent. Compositions for intravenous administration can optionally include a local anesthetic such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachet indicating the quantity of active agent. Where the active compound is to be administered by infusion, it can be dispensed, for example, with an infusion bottle containing sterile pharmaceutical grade water or saline. Where the active compound is administered by injection, an ampoule of sterile water for injection or saline can be provided so that the ingredients can be mixed prior to administration.

Compositions for oral delivery can be in the form of tablets, lozenges, aqueous or oily suspensions, granules, powders, emulsions, capsules, syrups, or elixirs, for example. Orally administered compositions can contain one or more optional agents, for example, sweetening agents such as fructose, aspartame or saccharin; flavoring agents such as peppermint, oil of wintergreen, or cherry; coloring agents; and preserving agents, to provide a pharmaceutically palatable preparation. Moreover, in tablet or pill form, the compositions can be coated to delay disintegration and absorption in the gastrointestinal tract providing a sustained action over an extended period of time. Selectively permeable membranes surrounding an osmotically active driving compound are also suitable for an orally administered of the active compound. In these later platforms, fluid from the environment surrounding the capsule is imbibed by the driving compound, which swells to displace the agent or agent composition through an aperture. These delivery platforms can provide an essentially zero order delivery profile as opposed to the spiked profiles of immediate release formulations. A time delay material such as glycerol monostearate or glycerol stearate can also be used. Oral compositions can include standard vehicles such as mannitol, lactose, starch, magnesium stearate, sodium saccharine, cellulose, magnesium carbonate, and the like. Such vehicles are preferably of pharmaceutical grade.

Further, the effect of the active compound can be delayed or prolonged by proper formulation. For example, a slowly soluble pellet of the active compound can be prepared and incorporated in a tablet or capsule. The technique can be improved by making pellets of several different dissolution rates and filling capsules with a mixture of the pellets. Tablets or capsules can be coated with a film that resists dissolution for a predictable period of time. Even the parenteral preparations can be made long-acting, by dissolving or suspending the compound in oily or emulsified vehicles which allow it to disperse only slowly in the serum.

Pharmaceutical compositions for use in accordance with the present invention can be formulated in conventional manner using one or more physiologically acceptable carriers or excipients.

Thus, the compound and optionally another therapeutic or prophylactic agent and their physiologically acceptable salts and solvates can be formulated into pharmaceutical compositions for administration by inhalation or insufflation (either through the mouth or the nose) or oral, parenteral or mucosal (such as buccal, vaginal, rectal, sublingual) administration. Local or systemic parenteral administration may be used.

For oral administration, the pharmaceutical compositions can take the form of, for example, tablets or capsules prepared by conventional means with pharmaceutically acceptable excipients such as binding agents (e.g., pregelatinized maize starch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulphate). The tablets can be coated by methods well known in the art. Liquid preparations for oral administration can take the form of, for example, solutions, syrups or suspensions, or they can be presented as a dry product for constitution with water or other suitable vehicle before use. Such liquid preparations can be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., almond oil, oily esters, ethyl alcohol or fractionated vegetable oils); and preservatives (e.g., methyl or propyl-p-hydroxybenzoates or sorbic acid). The preparations can also contain buffer salts, flavoring, coloring and sweetening agents as appropriate.

Preparations for oral administration can be suitably formulated to give controlled release of the active compound.

For buccal administration the pharmaceutical compositions can take the form of tablets or lozenges formulated in conventional manner.

For administration by inhalation, the pharmaceutical compositions for use according to the present invention are conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit can be determined by providing a valve to deliver a metered amount. Capsules and cartridges of e.g., gelatin for use in an inhaler or insufflator can be formulated containing a powder mix of the compound and a suitable powder base such as lactose or starch.

The pharmaceutical compositions can be formulated for parenteral administration by injection, e.g., by bolus injection or continuous infusion. Formulations for injection can be presented in unit dosage form, e.g., in ampoules or in multi-dose containers, with an added preservative. The pharmaceutical compositions can take such forms as suspensions, solutions or emulsions in oily or aqueous vehicles, and can contain formulatory agents such as suspending, stabilizing and/or dispersing agents. Alternatively, the active ingredient can be in powder form for constitution with a suitable vehicle, e.g., sterile pyrogen-free water, before use.

The pharmaceutical compositions can also be formulated in rectal compositions such as suppositories or retention enemas, e.g., containing conventional suppository bases such as cocoa butter or other glycerides.

In addition to the formulations described previously, the pharmaceutical compositions can also be formulated as a depot preparation. Such long acting formulations can be administered by implantation (for example subcutaneously or intramuscularly) or by intramuscular injection. Thus, for example, the pharmaceutical compositions can be formulated with suitable polymeric or hydrophobic materials (for example as an emulsion in an acceptable oil) or ion exchange resins, or as sparingly soluble derivatives, for example, as a sparingly soluble salt.

The pharmaceutical composition may be packaged in a hermetically sealed container such as an ampoule or sachet indicating the quantity. In one
examples, the pharmaceutical composition is supplied as a dry sterilized lyophilized powder or water free concentrate in a hermetically sealed container and can be reconstituted, e.g., with water or saline to the appropriate concentration for administration to a patient.

In other examples radiation therapy agents such as radioactive isotopes can be given orally as liquids in capsules or as a drink. Radioactive isotopes can also be formulated for intravenous injection. The skilled oncologist can determine the preferred formulation and route of administration.

The pharmaceutical compositions can, if desired, be presented in a pack or dispenser device that can contain one or more unit dosage forms containing the active ingredient. The pack can for example comprise metal or plastic foil, such as a blister pack. The pack or dispenser device can be accompanied by instructions for administration.

The pack or dispenser may contain one or more unit dosage forms containing no more than the recommended dosage formulation as determined in the Physician's Desk Reference (56^{th} ed. 2002).

Methods of administering the active compound and optionally another therapeutic or prophylactic agent include, but are not limited to, parenteral administration (e.g., intradermal, intramuscular, intraperitoneal, intravenous and subcutaneous), epidural, and mucosal (e.g., intranasal, rectal, vaginal, sublingual, buccal or oral routes). The active compound and optionally another prophylactic or therapeutic agents may be administered intramuscularly, intravenously, or subcutaneously. The active compound and optionally another prophylactic or therapeutic agent can also be administered by infusion or bolus injection and can be administered together with other biologically active agents. Administration can be local or systemic. The active compound and optionally the prophylactic or therapeutic agent and their physiologically acceptable salts and solvates can also be administered by inhalation or insufflation (either through the mouth or the nose). Local or systemic parenteral administration may be used.

It can be desirable to administer the active compound locally to the area in need of treatment. This can be achieved, for example, and not by way of limitation, by local infusion during surgery, topical application, e.g., in conjunction with a wound dressing after surgery, by injection, by means of a catheter, by means of a suppository, or by means of an implant, said implant being of a porous, non-porous, or gelatinous material, including membranes, such as silastic membranes, or fibers. Furthermore, administration can be by direct injection at the site (or former site) of inflamed tissue.

Pulmonary administration can also be employed, e.g., by use of an inhaler or nebulizer, and formulation with an aerosolizing agent, or via perfusion in a fluorocarbon or synthetic pulmonary surfactant. The active compound can be formulated as a suppository, with traditional binders and vehicles such as triglycerides.

The active compound can be delivered in a vesicle, in particular a liposome.

The active compound can be delivered in a controlled release system. A pump can be used. Furthermore, polymeric materials can be used.

The amount of the active compound that is effective in the treatment or prevention of heart conditions can be determined by standard research techniques. For example, the dosage of the active compound which will be effective in the treatment or prevention of heart conditions can be determined by administering the active compound to an animal in a model such as, e.g., the animal models known to those skilled in the art. In addition, in vitro assays can optionally be employed to help identify optimal dosage ranges.

Selection of a particular effective dose can be determined (e.g., via clinical trials) by a skilled artisan based upon the consideration of several factors which will be known to one skilled in the art. Such factors include the disease to be treated or prevented, the symptoms involved, the patient's body mass, the patient's immune status and other factors known by the skilled artisan.

The precise dose to be employed in the formulation will also depend on the route of administration, and the seriousness of the disease-related wasting, and should be decided according to the judgment of the practitioner and each patient's circumstances. Effective doses can be extrapolated from dose-response curves derived from in vitro or animal model test systems.

The dose of the active compound to be administered to a patient, such as a human, is rather widely variable and can be subject to independent judgment. It is often practical to administer the daily dose of the active compound at various hours of the day. However, in any given case, the amount of the active compound administered will depend on such factors as the solubility of the active component, the formulation used, patient condition (such as weight), and/or the route of administration.

The general range of effective amounts of the active compound alone or in combination with another prophylactic or therapeutic agent(s) are from about 0.001 mg/day to about 1000 mg/day, more preferably from about 0.001 mg/day to 750 mg/day, more preferably from about 0.001 mg/day to 500 mg/day, more preferably from about 0.001 mg/day to 250 mg/day, more preferably from about 0.001 mg/day to 100 mg/day, more preferably from about 0.001 mg/day to 75 mg/day, more preferably from about 0.001 mg/day to 50 mg/day, more preferably from about 0.001 mg/day to 25 mg/day, more preferably from about 0.001 mg/day to 10 mg/day, more preferably from about 0.001 mg/day to 1 mg/day. Of course, it is often practical to administer the daily dose of compound in portions, at various hours of the day. However, in any given case, the amount of compound administered will depend on such factors as the solubility of the active component, the formulation used, subject condition (such as weight), and/or the route of administration.

### EXAMPLES

### Example 1. Mutagenesis Analysis

Antisera to TLR3 and poly(I:C), were purchased from Imgenix Inc. (San Diego, CA). Dithiothreitol (DTT), iodoacetamide and trypsin were purchased from sigma Chemical Co. The water used in all procedures was purified using a Millipore Milli-Q UV plus purification system. All the organic solvents used for mass spectrometry were HPLC grade and all the other chemicals were reagent grade. The fluorescent dye that stains acidic membrane, Lysotracker, was purchased from Molecular Probes (Eugene OR). The 40 bp poly(I:C) was chemically synthesized. A 20-bp poly(I:C) was made by treating polyinosinic acid and polycytidylic acid with 1M NaOH for 5 minutes, and then separating the bands on a denaturing gel and cutting out the 20-nt bands and annealing them.

To obtain biochemical evidence for disulfide bonds in TLR3, we used mass spectrometry to examine the recombinant TLR3 ECD purified from human cells, named hTLR3ECD. One aliquot of hTLR3ECD was reduced and alkylated similar to protocols in Sechi, S., and Chait, B. T. Anal.Chem. 1998, 70, 5150-5158. The other aliquot was diluted with 10 uL of 50 mM ammonium bicarbonate, pH=8. Each aliquots was then thermally denatured at 90 °C for 15 min. The thermally denatured proteins were digested with sequencing grade modified trypsin at 37 °C for overnight. The molar ratio of trypsin to protein used is 1:40. Each sample was desalted using a C₁₈ Zip Tip (Millipore) before analysis by mass spectrometry (MS) utilizing an ABI 4700 Proteomics Analyzer (Applied Biosystems, Framingham, MA). 4-hydroxy-α-cyanocinnamicacid (5 mg/ml in 50% acetonitrile, 0.1% trifluoroacetic acid) was used as a matrix and mixed 1:1 with the desalted sample and spotted on the MALDI plate. All spectra were taken manually. For the tandem MS experiments the acceleration was set at 1 kV and the collusion gas was atmosphere.

The wild-type TLR3 plasmid was previously described in Sun et al. (2006). J. Biol. Chem. 281, 11144-11151. Site-directed mutations were made using oligonucleotides annealed to the target sequence and the QuickChange kit (Stratagene Inc., San Diego CA). Sequences of the oligonucleotides will be made available upon request. Several clones that resulted from the mutational analysis were sequenced to confirm the mutation. Mutant clones with affected activity were sequenced to confirm the presence of the mutation and the absence of unintended changes in the protein.

The model of the TLR3 ectodomain was based on the crystal structure determined by Bell et al. (2005) Proc Natl Acad Sci USA 102, 10976-10980. Two TLRECD molecules were docked into a dimer based on Bell et al. (2006). Proc Natl Acad Sci USA 103, 8792-8797. (13). The manual docking was performed in the Quanta molecular modeling environment (version 2000, Accelrys). The result was rendered using Pymol (version 0.99, DeLano Scientific LLC).

Cells were plated on LabTek II CC2 treated chamber slides (Nunc Intl., Naperville, IL) and transfected with plasmids in Lipofectamine2000 (Invitrogen, Carlsbad, CA). Each TLR3 mutant was visualized 24 hours post transfection with a Zeiss Axioplan fluorescent microscope via immunofluorescence. Briefly, the cells were removed from the incubator and rinsed with PBS before being fixed with 4% formaldehyde in PBS and permeabilized with 0.1% Triton X-100. The cells were then incubated at room temperature in the dark for at least 1 hour in anti- TLR3 FITC-conjugated monoclonal antibody (Imgenex315A San Diego, CA). The cells were washed and counterstained with Hoechst 33342 dye (Molecular Probes, Eugene, OR) before being mounted in a buffered glycerol aqueous mounting medium.

293T cells were transiently transfected with wild type TLR3, mutant TLR3 or control pcDNA as described above. Thirty-six hours post transfection, the cells were lysed using passive lysis buffer (Promega Inc.) and sonicated to degrade chromosomal DNA. Equal amounts of proteins from each sample were separated on NuPAGE 4-12% bis-tris gel (Invitrogen), blotted onto PVDF membrane and probed with anti- TLR3 MAb IMG315A (Imgenex Inc.). The blots were developed with peroxidase conjugated secondary antibodies and ECL-plus western blotting detection system (Amersham Biosciences).

FACS analyses were performed with 293T cells grown in 6-well collagen-coated plates (BD Biosciences) at a concentration of 2 x 10⁶ cells/well. The cells were transfected with 1 µg of the appropriate plasmids using Lipofectamine 2000. (Invitrogen Inc.). Eighteen to twenty-four hours after transfection, the cells were harvested and washed twice with ice-cold FACS buffer (1X PBS (10 mM Phosphate, 150 mM NaCl, pH7.4; + 3% fetal bovine serum + 0.04% sodium azide) before suspension at ∼2x10⁷ cells/mL in FACS buffer. The cells were stained for 30 minutes at 4°C with 1 µg PE-labeled anti-human TLR3 mAb (TLR3.7, purchased from eBioscience, San Diego; CA) or a negative control mouse IgG1 control antibody. The antibodies were added to cells grown in 96 well plates and incubated for 30 min on ice in the dark. The cells were washed twice with FACS buffer to remove unbound antibody, then resuspended in FACS buffer. Viaprobe (BD Biosciences) was used to exclude dead cells. The cells were transferred to the appropriate tubes and analyze using a FACS Calibur machine (BD Biosciences).

### Example 2.

Regulating TLR3 can modulate the inflammatory response that can prove deadly or debilitating in sepsis, arthritis, and asthma, to name only a few diseases. TLR3 needs to form homo-oligomers as part of the mechanism of action (MOA). This MOA suggests that mutant versions of TLR3 that can suppress signaling of the wild-type TLR3 (so called dominant negative mutants) can be used to modulate the inflammation response. Dominant negative TLR3 have been reported, including TLR3-DeltaTIR, which lacks the intracellular signaling domain of TLR3, indicating that the extracellular domain is required for dominant negativity (Ranjith-Kumar et al., 2007. J.B.C. 282, p. 7668). Other dominant negatives include mutations in the extracellular domain of TLR3 (ibid).

Surprisingly, we have identified a TLR3 mutant that lacks a substantial portion of the extracellular domain. Mutant TLR3N lacks residues 123-590 (deleting the motifs from LLR4 to part of LLR 22). Missing in TLR3N are Loop 2 and several of the previously identified residues demonstrated to be required for TLR3 dimerization (ibid). Analysis of TLR3N thus uncovered a second pathway for dominant negative inhibition of TLR3 signaling.

We expect that TLR3N can be targeted to the plasma membrane since it contains both the signal peptide as well as the N-terminus and C-terminus caps of TLR3 (Bell, J. K. et al., (2005) Proc. Natl. Acad. Sci. U.S. A. 102, 10976.

Dominant negativity assays have been conducted for TLR3N using reporter luciferase driven from either the NF-kB or the ISRE promoters. Renilla luciferase was used as a transfection control and all data is normalized as folds over the Renella control. The results are in agreement. In addition, TLR3N was compared with TLR3deltaTIR and its dominant negative activity was found to be comparable to that of TLR3delta-TIR.

TLR3 is an important regulator of the inflammation response. The discovery of a new dominant negative mutant TLR3 with a different MOA than previous mutants can form the basis of a new class of regulators of the inflammation response.

## Claims

1. A pharmaceutical composition comprising a TLR3 polypeptide comprising a mutation in Loop 2, wherein said polypeptide is a dominant negative inhibitor of a TLR protein.

2. The pharmaceutical composition of claim 1, wherein said inhibited TLR protein is wild-type TLR3 or TLR9.

3. The pharmaceutical composition of claim 1, wherein said polypeptide comprises the motif HANPGGPIY.

4. A pharmaceutical composition comprising a dominant negative TLR3 polypeptide comprising a mutation in Loop 2 for use in medicine.

5. A pharmaceutical composition comprising a nucleic acid sequence that encodes a dominant negative TLR3 amino acid sequence comprising a mutation in Loop 2 for use as a medicament for preventing or treating inflammatory diseases.

6. The composition for use according to claim 5, wherein said inflammatory disease is selected from the group consisting of pulmonary diseases, autoimmune diseases, fibrotic diseases, and kidney diseases.

7. The composition for use according to claim 6, wherein said pulmonary disease is selected from the group consisting of asthma, asthma exacerbation, microbial-associated pneumonia, sarcoidosis and cystic fibrosis; or wherein said autoimmune disease is selected from the group consisting of rheumatoid arthritis, psoriatic arthritis, and giant cell arthritis.

8. The composition for use according to claim 6, wherein said kidney disease is lupus nephritis; or wherein said fibrotic disease is liver fibrosis.

9. The composition for use according to any previous claim, further comprising administering a second therapeutic agent.

10. The composition for use according to claim 9, wherein said second therapeutic agent is selected from the group consisting of antimicrobial agents, corticosteroids and immuno-modulatory agents.

11. A method of inhibiting Toll-like receptor 3 activity comprising:
i) providing:
a) a cell comprising toll-like receptor 3 and
b) a dominant negative TLR3 amino acid sequence comprising a mutation in Loop 2, and
ii) mixing said cell and said amino acid sequence *in vitro* under conditions such that toll-like receptor 3 activity is inhibited.

12. The method of claim 11, wherein said amino acid sequence is a mutant of an amino acid sequence with a substituted or unsubstituted motif X¹X²NX⁴GGPX⁸X⁹ wherein X¹, X², X⁴, X⁸, and X⁹ are each individually and independently a naturally or non-naturally occurring amino acid.

13. The method of claim 12, wherein:
X¹ is H, R, or N;
X² is A or N;
X⁴ is P or V;
X⁸ is I or V;
and X⁹ is Y, H, Q, N, and M.

14. The method of claim 12, wherein said cells are selected from the group, HEK, HeLa, COS and Chinese Hamster Ovary cells.

## Patentansprüche

1. Pharmazeutische Zusammensetzung umfassend ein TLR3-Polypeptid, das eine Mutation in Schleife 2 umfasst, wobei besagtes Polypeptide ein dominant negativer Inhibitor eines TLR-Proteins ist.

2. Pharmazeutische Zusammensetzung nach Anspruch 1, wobei besagtes inhibiertes TLR-Protein Wildtyp von TLR3 oder TLR9 ist.

3. Pharmazeutische Zusammensetzung nach Anspruch 1, wobei besagtes Polypeptid das Motiv HANPGGPIY umfasst.

4. Pharmazeutische Zusammensetzung umfassend ein dominant negatives TLR3-Polypeptid, das eine Mutation in Schleife 2 umfasst, zur Verwendung in der Medizin.

5. Pharmazeutische Zusammensetzung umfassend eine Nukleinsäuresequenz, die eine dominant negative TLR3-Aminosäuresequenz kodiert, die eine Mutation in Schleife 2 umfasst, zur Verwendung als Medikament zum Schutz vor oder zur Behandlung von Entzündungserkrankungen.

6. Zusammensetzung zur Verwendung nach Anspruch 5, wobei besagte Entzündungserkrankung ausgewählt ist aus der Gruppe bestehend aus Lungenkrankheiten, Autoimmunkrankheiten, fibrotischen Erkrankungen und Nierenerkrankungen.

7. Zusammensetzung zur Verwendung nach Anspruch 6, wobei die besagte Lungenkrankheit ausgewählt ist aus der Gruppe bestehend aus Asthma, Asthma-Verschlimmerungen, Mikrobenassoziierter Pneumonie, Sarkoidose und zystischer Fibrose; oder wobei besagte Autoimmunkrankheit ausgewählt ist aus der Gruppe bestehend aus rheumatoider Arthritis, psoriatischer Arthritis und Riesenzellen-Arthritis.

8. Zusammensetzung zur Verwendung nach Anspruch 6, wobei besagte Nierenerkrankung Lupus nephritis ist; oder wobei besagte fibrotische Erkrankung Leberfibrose ist.

9. Zusammensetzung zur Verwendung nach einem der vorangehenden Ansprüche, des Weiteren umfassend die Verabreichung eines zweiten therapeutischen Mittels.

10. Zusammensetzung zur Verwendung nach Anspruch 9, wobei besagtes zweites therapeutisches Mittel ausgewählt ist aus der Gruppe bestehend aus antimikrobiellen Mitteln, Kortikosteroiden und immunmmodulatorischen Mitteln.

11. Verfahren zur Inhibierung der Aktivität des Toll-ähnlichen Rezeptors 3, umfassend:
i) die Bereitstellung:
a) einer Zelle umfassend einen Toll-ähnlichen Rezeptor 3 und
b) eine dominant negative TLR3-Aminosäurensequenz umfassend eine Mutation in Schleife 2, und
ii) Mischung besagter Zellen und besagter Aminosäurensequenz *in vitro* unter solchen Bedingungen, dass die Aktivität des Toll-ähnlichen Rezeptors 3 inhibiert wird.

12. Verfahren nach Anspruch 11, wobei besagte Aminosäuresequenz eine Mutante einer Aminosäurensequenz ist mit einem substituierten oder nicht substituierten Motiv X¹X²NX⁴GGPX⁸X⁹, wobei X¹, X², X⁴, X⁸ und X⁹ jeweils individuell und unabhängig eine natürliche oder nicht in der Natur vorkommende Aminosäure ist.

13. Verfahren nach Anspruch 12, wobei:
X¹ H, R oder N ist;
X² A oder N ist;
X⁴ P oder V ist;
X⁸ I oder V ist;
und X⁹ Y, H, Q, N und M ist.

14. Verfahren nach Anspruch 12, wobei besagte Zellen ausgewählt sind aus der Gruppe bestehend aus HEK-Zellen, HeLa-Zellen, COS-Zellen und chinesische Hamster-Ovarialzellen.

## Revendications

1. Composition pharmaceutique comprenant un polypeptide TLR3 comprenant une mutation dans la boucle 2, dans laquelle ledit polypeptide est un inhibiteur négatif dominant d'une protéine TLR.

2. Composition pharmaceutique selon la revendication 1, dans laquelle ladite protéine TLR inhibée est la protéine TLR3 ou TLR9 de type sauvage.

3. Composition pharmaceutique selon la revendication 1, dans laquelle ledit polypeptide comprend le motif HANPGGPIY.

4. Composition pharmaceutique comprenant un polypeptide TLR3 négatif dominant comprenant une mutation dans la boucle 2 pour une utilisation en médecine.

5. Composition pharmaceutique comprenant une séquence d'acides nucléiques qui code pour une séquence d'acides aminés TLR3 négative dominante comprenant une mutation dans la boucle 2 pour une utilisation en tant que médicament destiné à la prévention ou au traitement de maladies inflammatoires.

6. Composition pour une utilisation selon la revendication 5, dans laquelle ladite maladie inflammatoire est choisie dans le groupe constitué par les maladies pulmonaires, les maladies auto-immunes, les maladies fibreuses et les maladies rénales.

7. Composition pour une utilisation selon la revendication 6, dans laquelle ladite maladie pulmonaire est choisie dans le groupe constitué par l'asthme, une exacerbation d'asthme, la pneumonie microbienne, la sarcoïdose et la fibrose kystique ; ou dans laquelle ladite maladie autoimmune est choisie dans le groupe constitué par la polyarthrite rhumatoïde, l'arthrite psoriasique et l'arthrite à cellules géantes.

8. Composition pour une utilisation selon la revendication 6, dans laquelle ladite maladie rénale est la néphrite lupique ; ou dans laquelle ladite maladie fibreuse est la fibrose hépatique.

9. Composition pour une utilisation selon l'une quelconque des revendications précédentes, comprenant en outre l'administration d'un second agent thérapeutique.

10. Composition pour une utilisation selon la revendication 9, dans laquelle ledit second agent thérapeutique est choisi dans le groupe constitué par les agents antimicrobiens, les corticoïdes et les agents immuno-modulateurs.

11. Méthode d'inhibition de l'activité du récepteur de type Toll 3 consistant à :
i) utiliser
a) une cellule comprenant le récepteur de type Toll 3 et
b) une séquence d'acides aminés TLR3 négative dominante comprenant une mutation dans la boucle 2, et
ii) mélanger ladite cellule et ladite séquence d'acides aminés *in vitro* dans des conditions telles que l'activité du récepteur de type Toll 3 est inhibée.

12. Méthode selon la revendication 11, dans laquelle ladite séquence d'acides aminés est un mutant d'une séquence d'acides aminés avec un motif X^{I}X²NX⁴GGPX⁸X⁹ substituée ou non substituée dans laquelle X¹, X², X⁴, X⁸ et X⁹ sont chacun individuellement et indépendamment un acide aminé d'origine naturelle ou non naturelle.

13. Méthode selon la revendication 12, dans laquelle :
X¹ est H, R ou N ;
X² est A ou N ;
X⁴ est P ou V ;
X⁸ est I ou V ;
et X⁹ est Y, H, Q, N et M.

14. Méthode selon la revendication 12, dans laquelle lesdites cellules sont choisies dans le groupe constitué par les cellules HEK, HeLa, COS et d'ovaire de hamster chinois.
